# EUROPÄISCHE PATENTSCHRIFT

(11) **EP 3 538 016 B1**
(45) Veröffentlichungstag und Bekanntmachung des Hinweises auf die Patenterteilung: **30.06.2021**
(21) Anmeldenummer: 17797361.7
(22) Anmeldetag: 14.11.2017
(51) Int. Cl.: A61C 8/00, A61C 13/00

(54) **IMPLANTAT AUS FASERVERSTÄRKTEM KUNSTSTOFF**
IMPLANT MADE FROM FIBRE-REINFORCED PLASTIC
IMPLANT EN MATIÈRE PLASTIQUE RENFORCÉE DE FIBRES

(30) Priorität: 14.11.2016 EP 16198694
(43) Veröffentlichungstag der Anmeldung: 18.09.2019
(73) Patentinhaber: Schwitalla, Andreas, 10435 Berlin (DE); Müller, Wolf-Dieter, 12524 Berlin (DE)
(72) Erfinder: Schwitalla, Andreas, 10435 Berlin (DE); Müller, Wolf-Dieter, 12524 Berlin (DE)
(74) Vertreter: Hertin und Partner Rechts- und Patentanwälte PartG mbB
(86) Internationale Anmeldenummer: PCT/EP2017/079161
(87) Internationale Veröffentlichungsnummer: WO 2018/087383

(56) Entgegenhaltungen:
- EP-A1- 1 598 028
- EP-A1- 2 703 141
- WO-A1-2005/079696
- WO-A1-2007/025782
- DE-A1- 10 055 465
- DE-A1-102013 211 175
- US-A1- 2009 061 385
- US-B1- 6 193 516

## Beschreibung

### Einleitung

Die vorliegende Erfindung betrifft ein Implantat aus Kunststoff, dadurch gekennzeichnet, dass es einen mit gezielt multidirektional angeordneten Langfasern verstärkten thermoplastischen Kunststoff mit einem Elastizitätsmodul E von 10-70 GPa umfasst. Weiterhin betrifft die vorliegende Erfindung ein System zur Herstellung eines erfindungsgemäßen Implantats, umfassend eine Vorrichtung zur Erfassung von Patientendaten bzgl. der Umgebung, in die ein Implantat eingesetzt werden soll, ein Computerprogramm zur Erstellung eines Modells für ein individualisiertes Implantat auf Basis der erfassten Patientendaten, und eine Vorrichtung zur Herstellung des individualisierten Implantats auf Basis des berechneten Modells mittels 3D-Druck und/oder Lasersintern.

### Stand der Technik

Ein Implantat ist ein künstliches Material, das im Körper von Menschen oder Tieren eingepflanzt und mit Komponenten des Skeletts verbunden wird, und dort dauerhaft oder zumindest über einen längeren Zeitraum verbleiben soll. Implantate kommen zum Einsatz, wenn ein natürliches Gewebe oder natürliches Material, wie z.B. Zähne, Knochen oder Gelenke, seine Funktion aufgrund von Erkrankung, Verschleiß oder traumatischer Schädigung nicht mehr erfüllen kann. Spezielle Ausführungsformen von Implantaten umfassen beispielsweise Zahnimplantate, Implantate für die Osteosynthese wie z.B. Osteosyntheseplatten, Spine Cages sowie verschiedene Formen von Gelenkersatz wie beispielsweise Endoprothesen, insbesondere für Knie- und Hüftgelenke.

Zahnimplantate beschreiben künstliche Zahnwurzeln, die für die Fälle eingesetzt werden, bei denen der Zahn und/oder die Wurzel beschädigt oder erkrankt sind. Zahnimplantate weisen ein schraubenförmiges oder zylindrisches Design auf und werden in den Kieferknochen eingepflanzt, um verlorengegangene Zähne zu ersetzen. Das Zahnimplantat übernimmt prinzipiell die gleiche Funktion wie die eigene Zahnwurzel, da es direkt mit dem Knochen verwächst. Mit dem Implantat können im späteren Verlauf einer Behandlung Kronen oder Brücken aufgesetzt werden, die einen Zahnersatz darstellen.

Beim Zahnimplantat unterscheidet man den Implantatkörper, der in den Knochen eingebracht wird, und das Aufbauteil/Abutment, mit dessen Hilfe die Kronen oder Haltestrukturen für Prothesen am Implantatkörper befestigt werden. Hierbei differenziert man zwischen ein- oder mehrteiligen Implantaten, bei denen der Implantatkörper und das Aufbauteil getrennt sind. Es gibt jedoch auch Implantatsysteme, bei denen das Verbindungselement (Abutment) zum Zahnersatz in einem Stück mit dem Implantatkörper gearbeitet ist. Es ist auch möglich, den Zahnersatz direkt auf dem Implantatkörper zu verschrauben, wenn z.B. der Zahnersatz und das Abutment aus einem Bauteil gefertigt sind, wie es bei verschraubbaren Zahnkronen (verschraubte Implantatrestaurationen) der Fall ist. Des Weiteren können auch andere Komponenten am Implantatkörper befestigt werden, wie z.B. Druckknöpfe ("Locator"), Magnete oder Stegkonstruktionen.

Zur Herstellung von Implantaten werden heutzutage verschiedene Materialien aus den Gruppen der Metalle, Keramiken und Kunststoffe herangezogen. Allen gemeinsam ist die Zulassung zur Langzeitimplantation aufgrund ihrer nachgewiesenen Biokompatibilität. Implantate, die in knöchernen Strukturen des menschlichen Skelettes verankert werden, sind hohen mechanischen Belastungen ausgesetzt und müssen daher entsprechend hohe Elastizitätsmodule aufweisen und stabil sein. Der Elastizitätsmodul (auch Zugmodul, Zug-E-Modul, Elastizitätskoeffizient, Dehnungsmodul, E-Modul oder Youngscher Modul) ist ein Materialkennwert, der den Zusammenhang zwischen Spannung und Dehnung bei der Verformung eines festen Körpers bei linear-elastischem Verhalten beschreibt. Der Elastizitätsmodul wird mit E-Modul oder als Formelzeichen mit E abgekürzt und hat die Einheit einer mechanischen Spannung (Pascal/Pa). Der Betrag des Elastizitätsmoduls ist umso größer, je mehr Widerstand ein Material seiner elastischen Verformung entgegensetzt.

Bisher kamen deshalb hier vornehmlich Titan und Titanlegierungen, aber auch Keramiken wie Zirkonoxid zur Anwendung. Titan und Zirkonoxidkeramik weisen eine hohe Festigkeit und nur eine sehr geringe physikalische Elastizität auf. Ein sehr elastischer Gegenstand (mit hoher Elastizität) hat einen vergleichsweise niedrigen Elastizitätsmodul. Die E-Module von Titan und Zirkonoxid betragen 105 bzw. 220 Giga-Pascal (GPa).

Die Festigkeit eines Werkstoffes beschreibt die maximale Beanspruchbarkeit durch mechanische Belastungen, bevor es zu einem Versagen kommt, also einer unzulässigen und insbesondere einer bleibenden Verformung oder zum Bruch kommt. Die Festigkeit wird in mechanischer Spannung (Kraft pro Querschnittsfläche) angegeben. Im Gegensatz dazu bezeichnet Elastizität die Eigenschaft eines Körpers oder Werkstoffes, unter Krafteinwirkung seine Form zu verändern und bei Wegfall der einwirkenden Kraft in die Ursprungsform zurückzukehren.

Besteht das prothetische Material bzw. Implantat aus einem dieser Materialien, werden z.B. bei Zahnimplantaten die Kaukräfte, die bei Belastung auf die prothetischen Komponenten wirken, vom Implantat über eine starre, ankylotische Verbindung direkt auf den Kieferknochen übertragen. Dies entspricht nicht der natürlichen Belastung beim Kauen, da die natürliche Aufhängung des Zahns im Kiefer elastisch ist, wodurch eine gewisse Eigenbeweglichkeit der Zähne möglich ist. Durch diese natürliche Zahnaufhängung wird der zahntragende Knochen auf Zug belastet, wenn die Bindegewebsfasern der sog. Wurzelhaut (Desmodont) bei Kaubelastung gestrafft werden.

Bei der Belastung eines Implantates aus Titan oder Zirkonoxid wird aufgrund der starren Verankerung der Knochen auf Druck belastet. Bei einer Überbelastung können demnach Stressspitzen im Implantat-Knocheninterface auftreten, welche den Knochen schädigen und demzufolge einen Knochenabbau nach sich ziehen können.

Zudem kann es auch unter Verwendung von sehr steifen Materialien für ein Implantat zu einem sogenannten "stress-shielding" Effekt kommen. Unter "stress-shielding" versteht man eine Verminderung der Knochendichte als Folge des Ausbleibens der normalen Belastung des Knochens durch die Überbrückung eines Implantats. Aufgrund der hohen Steifigkeit eines Implantats werden die normalen Beanspruchungen allein durch das Implantat getragen, wobei der benachbarte Knochen übermäßig geschont wird. Die Verwendung von Implantaten mit sehr hoher Steifigkeit, wie z.B. Implantate aus Titan oder Zirkonoxid, führen daher in manchen Bereichen zu einem pathologischen Umbau des an das Implantat angrenzenden Knochengewebes, was dort mit einem Verlust an Dichte und Festigkeit des Knochens einhergeht.

Aufgrund ihrer hohen Steifigkeit und der damit einhergehenden geringen Elastizität besteht zudem eine hohe Bruchgefahr für Implantate aus Titan und Zirkonoxid bei hoher mechanischer Belastung.

Titan hat außerdem den Nachteil, dass es eine hohe Plaqueaffinität hat, was insbesondere bei der Verwendung als Zahnimplantat nachteilig ist. Zudem ist es nicht möglich, eine bakteriendichte Verbindung zwischen einem Zahnimplantat aus Titan und den verwendeten prothetischen Aufbauten herzustellen. Weiterhin legen Patienten insbesondere bei Zahnimplantaten inzwischen Wert auf eine metallfreie Versorgung, auch aufgrund der dunklen Eigenfarbe von Titan. Ein weiterer Nachteil von Titanimplantaten ist, dass manche Patienten überempfindlich gegen Titan sind, weshalb in diesen Fällen eine Verwendung von Titanimplantaten nicht möglich ist.

Daher werden neben den sehr steifen Materialien Titan und Zirkonoxid zunehmend Hochleistungskunststoffe, wie beispielsweise Polyetheretherketon (PEEK) zur Herstellung von Implantaten verwendet. Da nach dem Einbringen in den Körper sehr hohe mechanische Belastungen auf Implantate wirken, insbesondere im Bereich der Verankerungsstrukturen, müssen die zur Herstellung von Implantaten genutzten Hochleistungskunststoffe jedoch häufig mit verstärkenden Zusätzen wie beispielsweise Fasern, insbesondere Kohlefasern, versetzt werden.

Knochen stellt ein anisotropes Material dar, einerseits aufgrund seiner unterschiedlich dicht ausgebildeten mineralisierten Strukturen, sowohl im kortikalen als auch insbesondere im spongiösen Bereich, und andererseits aufgrund der funktionellen Anpassung beispielsweise an die Trajektorien, die den größten Druck- und Zugbelastungen des Knochens entsprechen. Gleichzeitig variieren die mechanischen Eigenschaften des Kieferknochens interindividuell sehr stark. Deshalb kann es auch bei Verwendung von faserverstärkten Implantate mit besonders hohen E-Modulen zu stress-shielding Effekten und/oder Überbelastung des Knochens kommen. Umgekehrt kann ein zu wenig faserverstärktes Implantat insbesondere im kortikalen Knochenbereich auch eine zu hohe Elastizität bzw. zu geringe Versteifung aufweisen, was ebenfalls zu einem pathologischem Knochenumbau führen kann.

Im Stand der Technik wurden mehrere Implantate beschrieben, die aus zwei oder mehr Materialien bestehen, die verschiedene Härtegrade oder Elastizitätsmodule aufweisen, um den angrenzenden Knochen zu schonen. Hierbei wird zumeist innen ein härteres Material angeordnet, und die Außenschicht besteht aus einem elastischeren Material.

US 6,193,516 B1 beschreibt Implantate, die einen metallischen Kern und eine aus Kunststoff gefertigte außen liegende Hülle umfassen, wobei es sich bei dem Kunststoff um mit kontinuierlichen Carbon Fasern verstärktes PEEK handeln kann. Es wird beschrieben, dass durch die Länge und Orientierung der Fasern die Materialeigenschaften des Kunststoffs angepasst werden können. Die in US 6,193,516 B1 beschriebenen Implantate unterscheiden sich von den Implantaten der vorliegenden Erfindung insbesondere dadurch, dass sie einen metallischen Kern enthalten und aus mehreren Bauteilen bestehen. Zudem wird nicht spezifiziert, um welche Art Fasern verwendet wird, insbesondere bzgl. der Faserdicke.

US 2009/061385 A1 beschreibt faserverstärkte Dentalimplantate aus PEEK, die aus verschiedenen miteinander verbunden Bauteilen aus unterschiedlichen Materialien mit unterschiedlichen Härtegrade bestehen. Es wird jedoch nicht beschrieben, dass die Fasern innerhalb des Implantats oder der einzelnen Bauteile des Implantats eine bestimmte Orientierung haben. Eine gezielte Anordnung der Fasern innerhalb des Kunststoffs ist nicht beschrieben. Der faserverstärkte Kunststoff, aus dem das Implantat besteht, wird lediglich über das Elastizitätsmodul definiert. Zudem werden die Fasern bzgl. ihrer Eigenschaften, wie z.B. der Dicke, nicht näher definiert. Daher unterscheiden sich diese Implantate grundlegend von den hier beschriebenen Implantaten der vorliegenden Erfindung, die aufgrund spezifischer Faseranordnung und Faserdicken überraschende Vorteile gegenüber den bekannten Implantaten aufweisen.

In DE 102013211175 A1 wurde vorgeschlagen die mechanische Belastung für den angrenzenden Knochen durch Implantate, die aus zwei miteinander verbundenen Kunststoffen-Komponenten gefertigt sind, zu reduzieren, wobei eine erste Kunststoff-Komponente mit einem E-Modul ähnlich dem von Knochen außen liegt, und eine zweite innenliegende Kunststoff-Komponente einen höheren E-Modul aufweist und dem Implantat die nötige Stabilität verleiht. Es wurde vorgeschlagen, dass die Implantate teilweise aus mit Langfasern verstärktem PEEK mit einem Elastizitätsmodul von 20-80 GPa gefertigt werden. Zudem werden in DE102013211175 keine Langfasern mit spezifischer Faserdicke beschrieben. Auch wird lediglich eine parallele Anordnung von Langfasern offenbart, die sich von der multidirektionalen Anordnung der Fasern in den hier beschriebenen erfindungsgemäßen Implantaten unterscheidet.

EP 1 598 028 A1 beschreibt ein Implantat, das eine äußere Hülle aus Metall und einen Kern aus Kunststoff, z.B. faserverstärktem PEEK, umfasst. Das Plastikbauteil des Implantats kann dabei mit Hilfe des CFM-Verfahrens hergestellt werden. Auch hier werden keine multidirektionale Faseranordnung und insbesondere keine gezielte multidirektionale Faseranordnung beschrieben. Zudem werden die Fasereigenschaften, wie z.B. die Faserdicke, nicht beschrieben.

DE 10055465 A1 beschreibt Knochenersatzwerkstoffe u.a. aus PEEK, deren E-Modul durch den Zusatz faserförmiger Füllstoffpartikel wie z.B. Fasern oder Kugeln mit einer Partikelgröße von 0,1-200 µm angepasst werden kann. Hieraus geht hervor, dass nur eine Verstärkung mit Kurzfasern offenbart wird. Zudem wir die Herstellung solcher Implantate durch Lasersintern mit Hilfe eines CAD/CAM Systems beschrieben. Es werden aber keine mit Langfasern verstärkte Werkstoffe beschrieben, insbesondere nicht solche, in denen die Langfasern multidirektional angeordnet sind und einen spezifischen Durchmesser haben, damit eine besonders gute Funktionalität erreicht wird. Zudem haben die Materialien gemäß DE 10055465 A1 ein niedrigeres E-Modul als der Kunststoff des hier beschriebenen erfindungsgemäßen Implantats.

In Bezug auf die im Stand der Technik beschriebenen Implantate ist festzustellen, dass diese alle aus mehreren verschiedenen Materialien oder Bauteilen hergestellt werden, die eine äußere Hülle und einen Kern umfassen. Meist ist zudem eine Komponente aus Metall gefertigt. Es hat sich zudem gezeigt, dass diese Implantate, und insbesondere die Implantate gemäß DE 102013211175 A1, entgegen des ursprünglichen Zwecks, nicht zu einer Schonung des Implantat-Knochen-Interface beitragen. Im Gegenteil, es zeigte sich, dass die erhöhte Elastizität der äußeren Schicht am Implantat-Knochen-Interface entsprechender Implantate eine nachteilige Lastenverteilung auf den umliegenden Knochen zur Folge hat, was zu einer Schädigung des Knochens und einer bindegewebigen Einscheidung des Implantates führt.

### Aufgabe der Erfindung

Der vorliegenden Erfindung liegt somit die Aufgabe zugrunde, ein Implantat bereitzustellen, das die oben beschriebenen Nachteile des Standes der Technik nicht aufweist. Entsprechend bestand die Aufgabe darin, ein Implantat bereitzustellen, das im wesentlichen metallfrei ist, die nötige Stabilität aufweist, um den mechanischen Belastungen, die auf ein Implantat nach dem Einsetzten in den Körper wirken, standzuhalten, eine geringe Bruchgefahr und eine niedrige Plaqueaffinität aufweist, eine bakteriendichte Verbindung der Implantatbauteile ermöglicht und nachteilige mechanische Belastungen des angrenzenden Knochens, wie beispielsweise stress-shielding Effekte und Überbelastungen, minimiert.

### Allgemeine Beschreibung der Erfindung

Die Erfindung ist in den Ansprüchen 1 bis 3 definiert.

Erfindungsgemäß wird die vorliegende technische Aufgabe durch die Bereitstellung eines Implantats aus Kunststoff gelöst, das einen faserverstärkten thermoplastischen Kunststoff mit einem Elastizitätsmodul E von 10-70 GPa umfasst.

In einer bevorzugten Ausführungsform betrifft die Erfindung ein Implantat aus Kunststoff, dadurch gekennzeichnet, dass es einen mit gezielt multidirektional angeordneten Langfasern verstärkten thermoplastischen Kunststoff mit einem Elastizitätsmodul E von 10-70 GPa umfasst.

Es war völlig überraschend, dass ein Implantat aus einem faserverstärkten thermoplastischen Kunststoff mit einem Elastizitätsmodul von 10-70 GPa eine nachteilige mechanische Belastung des angrenzenden Knochens im Vergleich zu bekannten Lösungen vermeidet. So treten bei Verwendung eines erfindungsgemäßen Implantats praktisch keine stress-shielding Effekte auf. Dies wird dadurch gewährleistet, dass erfindungsgemäße Implantate aufgrund ihres Elastizitätsmoduls den mechanischen Belastungen zwar standhalten können, jedoch diese nicht unverändert auf den angrenzenden Knochen weiterleiten, wie dies bei den deutlich steiferen Implantat-Lösungen aus Titan und Keramik der Fall ist. Vielmehr kann ein erfindungsgemäßes Implantat aus faserverstärktem thermoplastischem Kunststoff die einwirkende Kraft durch seine Elastizität teilweise absorbieren.

Andererseits kommt es auch nicht zu einer unnatürlichen Belastung des Knochens, wie sie bei Implantaten aus thermoplastischen Kunststoffen ohne Faserverstärkung vorkommt, die aufgrund ihrer zu hohen Elastizität zu einem unphysiologischen bzw. pathologischen Umbau des angrenzenden Knochens führen. Überraschenderweise ist bei faserverstärkten thermoplastischen Kunststoffen mit einem Elastizitätsmodul von 10-70 GPa die Balance zwischen Steifigkeit und Elastizität nahezu ideal im Vergleich zu bekannten Implantatlösungen.

Aufgrund dieser überraschenden mechanischen Eigenschaften eines erfindungsgemäßen Implantats werden ähnliche mechanische Kräfte auf den angrenzenden Knochen übertragen, wie dies unter physiologischen Bedingungen, beispielsweise bei der Kraftübertragung durch einen gesunden Zahn und die natürliche Zahnaufhängung der Fall ist.

Das Implantat weist zudem die nötige Festigkeit auf, um den unter normalen und hohen Belastungen im Körper auf das Implantat wirkenden Kräften standhalten zu können. Gleichzeitig hat es die notwendige Elastizität und Flexibilität, um auch bei sehr hoher mechanischer Belastung nicht zu brechen.

Diese oben beschriebenen vorteilhaften mechanischen Eigenschaften und alle anderen hier genannten Vorteile der Erfindung betreffen insbesondere erfindungsgemäße Implantate mit Langfaserverstärkung und einem Elastizitätsmodul von 30-50 GPa. Überraschenderweise sind die Vorteile besonders ausgeprägt, wenn die Langfasern einen Durchmesser von 4-10 µm haben und/oder multidirektional oder gezielt multidirektional angeordnet sind.

Zudem hat sich überraschender Weise gezeigt, dass die hierin genannten vorteilhaften mechanischen Eigenschaften von erfindungsgemäßen Implantaten dadurch noch weiter verstärkt werden können, dass die Implantate aus nur einem Bauteil bzw. einem einheitlichen Material hergestellt werden.

Gemäß einer bevorzugten Ausführungsform besteht das erfindungsgemäße Implantat aus einem einzelnen Bauteil. Im Sinne der Erfindung bedeutet "einzelnes Bauteil", dass das Implantat in einem einheitlichen Verfahren gefertigt wird und nicht zunächst einzelne Teile des Implantats hergestellt werden, die miteinander verbunden werden. Bevorzugt besteht das erfindungsgemäße Implantat somit aus nur einem Material. Dieses Material kann zwar lokal unterschiedliche Compoundzusätze oder Faseranordnungen aufweisen, es handelt sich aber um ein in einem einheitlichen Verfahren hergestelltes Material.

Als Bauteile im Sinne der Erfindung verstanden werden insbesondere ein Implantat-Kern und eine umgebende Implantat-Hülle oder -Schale, die aus unterschiedlichen Materialien mit unterschiedlichen (mechanischen) Eigenschaften bestehen können.

Hierin unterscheidet sich diese Ausführungsform des erfindungsgemäßen Implantats von Implantaten des Standes der Technik, die aus einem Kern und einer Schale bestehen und/oder aus unterschiedlichen Materialien mit unterschiedlichen mechanischen Eigenschaften zusammengesetzt sind und nach Fertigung der Einzelmaterialien miteinander verbunden werden. Die Idee bei der Entwicklung solcher Implantate aus mehreren Materialien und/oder Bauteilen mit unterschiedlichen mechanischen Eigenschaften war, dass hierdurch ggf. die Belastung für den angrenzenden Knochen reduziert werden könnte. Diese Hypothese hat sich aber bisher nicht bewahrheiten können und es wurde festgestellt, dass insbesondere Implantate, deren Schale/Außenhülle eine im Vergleich zum Kern generell oder pauschal verringerte Härte/Elastizität aufweisen, zu einer erhöhten Knochenschädigung führen.

Im Gegensatz dazu zeigte sich im Rahmen der vorliegenden Erfindung, dass negative Knochenumbaueffekte besonders stark reduziert werden und teilweise fast gar nicht auftreten, wenn erfindungsgemäße Implantate aus nur einem Bauteil verwendet werden, die keine pauschal unterschiedliche Kern und Schale aufweisen. Vielmehr hat sich gezeigt, dass bereichsweise Modifikationen der Compoundierung, insbesondere durch multidirektionale (gezielte) Faseranordnung innerhalb eines ansonsten homogenen einzelnen Bauteils, das nicht in Schale und Kern oder andere Bestandteile unterteilt ist, besonders positive Auswirkungen auf den Erhalt und die Stabilität des angrenzenden Knochen haben.

Ein großer Vorteil der erfindungsgemäßen Implantate aus Kunststoff im Vergleich zu Implantaten auf Titanbasis ist, dass diese nicht korrodieren und somit kein korrosionsbedingtes, erhöhtes Bruchrisiko aufweisen.

Im Vergleich zu bekannten Kunststoffimplantaten mit einer höheren Elastizität haben erfindungsgemäße Implantate den großen Vorteil, dass sie unter den mechanischen Belastungen im Körper weniger verformen. Die elastischen Verformungen führen bei bekannten Kunststoffimplantaten dazu, dass die Implantatoberfläche porös wird, also kleine Risse, Vertiefungen und Hohlräume entwickeln, die die Besiedelung durch Mikroorganismen wie beispielsweise Bakterien und Pilzen erleichtert, was zu Entzündungen des umgebenden Gewebes führt und auch die Gefahr von weitreichender gesundheitlicher Beeinträchtigung des Implantatträgers durch systemische Ausbreitung der Infektion birgt. Diese Gefahr ist durch die spezifischen elastischen Eigenschaften von erfindungsgemäßen Implantaten nicht vorhanden, da die elastischen Verformungen, die aufgrund des spezifischen E-Moduls ermöglicht werden, nicht so weitreichend sind, dass die Implantatoberfläche porös werden würde.

Ein überraschender Vorteil des erfindungsgemäßen Implantats ist, dass es sich unter sehr hohen Belastungen geringfügig biegen oder verformen lässt, wodurch ein Bruch des Implantats vermieden wird. Gleichzeitig wird hierdurch ein Teil der auf das Implantat einwirkenden Kraft absorbiert und nicht direkt weiter auf den Knochen übertragen, was vorteilhaft für die mechanische Belastung des angrenzenden Knochens ist. Hierbei ist es besonders vorteilhaft, dass diese Verbiegungen oder Verformungen komplett reversibel sind und die erfindungsgemäßen Implantate nach Beendigung der Krafteinwirkung in ihren Ausgangszustand zurückversetzt werden, ohne dass dabei Funktionseinbußen auftreten.

Dies ist ein Vorteil gegenüber bekannten Implantaten aus Kunststoff, die sich bei hohen mechanischen Belastungen aufgrund ihrer im Vergleich zu den erfindungsgemäßen Implantaten höheren Elastizität stärker Verformen, was zu irreversiblen Verformungen und zum Versagen des Materials führen kann. Erfindungsgemäße Implantate haben daher deutliche längere Gebrauchszeiten als bekannt Kunststoffimplantate, die regelmäßig ausgetauscht werden müssen, da sie den mechanischen Belastungen nicht dauerhaft standhalten können.

Zudem ermöglicht ein erfindungsgemäßes Implantat aus faserverstärktem thermoplastischem Kunststoff eine metallfreie Versorgung des Patienten. Aufgrund der spezifischen elastischen Eigenschaften eines erfindungsgemäßen Implantats mit einem E-Modul von 10-70 GPa wird auch unter funktioneller Belastung eine bakteriendichte Verbindung zu anderen prothetischen Komponenten ermöglicht. Dies ist mit bekannten Implantaten aus Kunststoff nicht möglich, da diese zu elastisch sind und auf Dauer den funktionellen Belastungen nicht standhalten, was zu einer Undichtigkeit der Verbindung führt.

Eine bakteriendichte Verbindung ist insbesondere bei zweiteiligen Zahnimplantaten, bestehend aus einem Implantatkörper, der die Funktion der Zahnwurzel übernimmt und im Knochen verankert ist, und einem Abutment, das als Platzhalter durch die Gingiva und als Träger der prothetischen Krone fungiert, von großer Wichtigkeit. Im Falle von miteinander verbundenen metallischen oder keramischen Bauteilen ist eine bakteriendichte Verbindung zwischen den Komponenten, wie auch immer diese geometrisch gestaltet sind, nicht zu erreichen. Die verbleibenden Spalten sind bakteriendurchlässig. So können beispielsweise Bakterien, die während der Insertion des Implantates in den inneren Schraubenkanal des Implantatkörpers gelangt sind, von dort aus auch nach Verschluss des Schraubenkanals durch die Verbindung mit dem Abutment nach außen gelangen und im Grenzbereich zwischen Knochen, Implantat und Schleimhaut Entzündungen hervorrufen.

Bei mehrteiligen Implantatvarianten aus Titan kommt es insbesondere bei horizontal einwirkenden Kräften zu einem Auseinanderklaffen des Implantat-Abutment-Interfaces. Dies bewirkt eine Art Pumpeffekt, der die bakterielle Besiedlung des Spaltes zwischen Implantatkörper und Abutment begünstigt und Exotoxine in das umliegende Gewebe drückt.

Der Begriff "Implantat" bezieht sich im Rahmen der vorliegenden Erfindung auf ein im Körper eines Menschen oder eines Tiers eingepflanztes künstliches Material, das dort mit Komponenten des Skeletts verbunden wird. Spezielle Ausführungsformen von Implantaten im Sinne der vorliegenden Erfindung umfassen, ohne Limitierung, Zahnimplantate, Implantate für die Osteosynthese wie beispielsweise Osteosyntheseplatten, Spine Cages (auch interbody fusion cages genannt), Nahtanker, verschiedene Formen von Gelenkersatz wie beispielsweise Endoprothesen, insbesondere Hüft-, Schulter- und Kniegelenksprothesen, sowie Prothesen zur Überbrückung von Knochendefekten, beispielsweise im Kopf- und Gesichtsbereich.

Der Begriff "Zahnimplantat" umfasst, ohne hierauf limitiert zu sein, Sofortimplantate, die eine anatomische Form entsprechend dem vorhandenen Zahnfach bzw. der Wurzel des entfernten Zahnes haben, und Spätimplantate, die einen runden Querschnitt entsprechend dem Durchmesser des Bohrers zum Anlegen des Implantatbetts haben. Sofortimplantate und Spätimplantate können jeweils als einteilige Zahnimplantate gefertigt sein, die einen Implantatkörper, ein Abutment und eine Zahnkrone in einem Bauteil umfassen. Alternativ können sie jeweils als einteilige Zahnimplantate gefertigt sein, die einen Implantatkörper und ein Abutment in einem Bauteil umfassen. Zudem können Sofortimplantate und Spätimplantate jeweils als zweiteilige Zahnimplantate, bei denen Implantatkörper und Abutment separate Bauteile sind, gefertigt sein. Weiterhin umfasst der Begriff Zahnimplantat, ohne Limitierung, Mini-Implantate, Hohlzylinderimplantate, Blattimplantate, Schmalkieferimplantate, Diskimplantate und subperiostale Implantate.

Im Zusammenhang mit der vorliegenden Erfindung ist unter dem Begriff "Kunststoff" ein im Wesentlichen aus Makromolekülen bestehender Werkstoff zu verstehen. Die jeweiligen Makromoleküle eines Kunststoffes sind Polymere, die aus sich wiederholenden Grundeinheiten aufgebaut sind. Kunststoffe werden bezüglich ihrer physikalischen Eigenschaften in drei großen Gruppen unterteilt, nämlich Thermoplaste, Duroplaste und Elastomere.

Thermoplastische Kunststoffe, oder Thermoplasten, sind Kunststoffe, die sich in einem bestimmten Temperaturbereich verformen lassen. Es ist vorteilhaft, dass das erfindungsgemäße Implantat aus einem solchen Kunststoff geformt ist, da sich somit Anpassungen in der Form und Beschaffenheit des Implantats durch Erhitzen des Materials erzielen lassen, so dass ggf. notwendige Änderungen an der Form auch kurz vor dem Einsetzen vorgenommen werden können. Auch ist es möglich, dass sich die Form des Implantats beim Einsetzen an die spezifische Umgebung anpasst, wenn es im erwärmten Zustand eingesetzt wird.

Ein weiterer Vorteil ergibt sich daraus, dass sich thermoplastische Kunststoffe durch Hitzeeinwirkung im 3D-Druckverfahren verarbeiten lassen. Dieses Verfahren gewährleistet eine freie Gestaltung der äußeren Form, unabhängig von aufwendig herzustellenden Spritzgussformen.

Thermoplastische Kunststoffe lassen sich bezüglich mechanischer, thermischer und chemischer Eigenschaften in die Gruppen Standardkunststoffe, technische Kunststoffe und Hochleistungskunststoffe einteilen. Zu den Thermoplasten zählen, ohne Limitierung, Acrylnitril-Butadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK), Polyetherketonketon (PEKK), Polyoxymethylen (POM), Polyvinylacetat (PVAC), thermoplastisches Polyuretan (TPU), Polyaryletherketone (PAEK) und Polyvinylchlorid (PVC).

Ein entscheidender Vorteil des erfindungsgemäßen Implantats ist es, dass durch die Faserverstärkung die mechanischen Eigenschaften des Implantats, wie beispielsweise die Steifigkeit und die Elastizität, zielgerichtet an die individuellen mechanischen Erfordernisse angepasst werden können.

Unter faserverstärkten Kunststoffen (auch als Faser-Kunststoff-Verbunde bezeichnet) sind Werkstoffe zu verstehen, die sowohl eine Kunststoffmatrix als auch verstärkende Fasern umfassen. Die Matrix umgibt die Fasern, die durch Adhäsiv- oder Kohäsivkräfte an die Matrix gebunden sind. Durch die Verwendung von Faserwerkstoffen haben Faser-Kunststoff-Verbunde ein richtungsabhängiges Elastizitätsverhalten. Ohne Matrixwerkstoff sind die hohen spezifischen Festigkeiten und Steifigkeiten der Verstärkungsfasern nicht nutzbar. Erst durch die geeignete Kombination von Faser- und Matrixwerkstoff entsteht ein neuer Konstruktionswerkstoff. Durch gegenseitige Wechselwirkungen der beiden Komponenten erhält dieser Werkstoff höherwertige Eigenschaften als jede der beiden einzeln beteiligten Komponenten. Die mechanischen und thermischen Eigenschaften von Faser-Kunststoff-Verbunden können unter anderem eingestellt werden über die Auswahl der Faser-Matrix-Kombination, den Faserwinkel, den Faservolumenanteil, die Schichtreihenfolge, die Faserorientierung oder die Faserlänge.

Verstärkungsfasern gemäß der hier offenbarten Erfindung umfassen, ohne Limitierung, Basaltfasern, Borfasern, Glasfasern, Keramikfasern, Kieselsäurefasern, Stahlfasern, Aramidfasern, Kohlenstofffasern, Polyesterfasern, Nylonfasern, Polyethylenfasern, Zirkonoxidfasern, Aluminiumoxidfasern, Siliziumcarbidfasern und Plexiglasfasern.

Das Elastizitätsmodul (auch Zugmodul, Elastizitätskoeffizient, Dehnungsmodul, E-Modul oder Youngsches Modul) ist ein Materialkennwert aus der Werkstofftechnik, der den Zusammenhang zwischen Spannung und Dehnung bei der Verformung eines festen Körpers bei linear-elastischem Verhalten beschreibt. Der Begriff Elastizitätsmodul wird mit E-Modul oder als Formelzeichen mit E abgekürzt und hat die Einheit einer mechanischen Spannung. Der Betrag des Elastizitätsmoduls ist umso größer, je mehr Widerstand ein Material seiner elastischen Verformung entgegensetzt. Ein Bauteil aus einem Material mit hohem Elastizitätsmodul (z. B. Titan) ist also steifer als ein Bauteil gleicher Konstruktion (also mit den gleichen geometrischen Abmessungen), welches aus einem Material mit niedrigem Elastizitätsmodul (z. B. Gummi) besteht.

Im Sinne der vorliegenden Erfindung ist es zudem zweckmäßig, wenn es sich bei den verstärkenden Fasern um Langfasern handelt. In einer weiteren bevorzugten Ausführungsform der Erfindung sind die verstärkenden Fasern Endlosfasern.

Vorteilhafterweise hat sich bei den Implantaten gemäß der Erfindung gezeigt, dass sich mit steigender Länge der verstärkenden Fasern auch die Steifigkeits- und Festigkeitswerte der erfindungsgemäßen Implantate aus faserverstärktem Kunststoff erhöhen. Entsprechend erzielen Kunststoffe, die Langfasern (> 1 mm Länge) enthalten, höhere Steifigkeits- und Festigkeitswerte als Kunststoffe, die Kurzfasern (< 1 mm Länge) enthalten. Im Sinne der vorliegenden Erfindung umfasst der Begriff Langfaser auch Endlosfasern mit einer Länge von > 50 mm. Endlosfasern sind somit eine besondere Ausführungsform von Langfasern.

Durch die Verstärkung erfindungsgemäßer Implantate mit Endlosfasern lassen sich besonders hohe Steifigkeits- und Festigkeitswerte erzielen. Entsprechend sind mit Lang- und insbesondere Endlosfasern verstärkte erfindungsgemäße Implantate Implantaten ohne Faserverstärkung oder solchen mit einer Kurzfaserverstärkung überlegen.

Zur Herstellung des erfindungsgemäßen Implantats werden bevorzugt Langfasern, insbesondere Endlosfasern eingesetzt. Die bisher üblichen und gängigen Verfahren zur Verarbeitung von Kunststoffen, die Lang- oder Endlosfasern enthalten, eignen sich nur bedingt zur Herstellung von Implantaten im Sinne der vorliegenden Erfindung. Diese Verfahren verwenden zumeist ein Halbzeug als Ausgangsmaterial, das durch ein Pultrusionsverfahren hergestellt worden ist und somit parallel angeordnete Fasern enthält, und aus dem Implantate durch Spanabtrag oder Fräsen hergestellt werden.

So hergestellte Implantate sind nur in beschränktem Maße geeignet, den mechanischen Kräften dauerhaft standzuhalten, die aus vielen unterschiedlichen Richtungen und mit unterschiedlichen Orientierungen beim oder nach dem Einsetzen in den Körper auf das Implantat einwirken, da sie nur unidirektional orientierte Fasern enthalten, die durch den Herstellungsprozess der Implantate aus dem Halbzeug zudem durchtrennt wurden. Beispiele für entsprechende Krafteinwirkungen sind das Anziehen von Befestigungsschrauben oder im Fall von Zahnimplantaten die einwirkenden Kaukräfte, die bei der Zerkleinern von Nahrungsmitteln auf das Implantat wirken. Es war daher völlig überraschend, dass es erfindungsgemäß gelungen ist, Implantate aus mit Lang- und/oder Endlosfasern verstärkten thermoplastischen Kunststoffen herzustellen, die diesen mechanischen Belastungen standhalten.

Eine weitere Ausführungsform des Implantats ist dadurch gekennzeichnet, dass die Fasern multidirektional oder unidirektional angeordnet sind.

Eine besonders bevorzugte Ausführungsform des Implantats ist dadurch gekennzeichnet, dass die Fasern gezielt multidirektional oder zufällig multidirektional angeordnet sind.

Eine "unidirektionale" Faserausrichtung bedeutet im Rahmen der vorliegenden Erfindung, dass alle Fasern, die in einem erfindungsgemäßen Implantat enthalten sind, die gleiche, parallele Ausrichtung haben. Hierdurch wird eine besonders hohe Stabilität und Zugfestigkeit des Implantats gegenüber Kräften erzielt, die entlang der Faserausrichtung auf das Implantat einwirken.

Im Sinne der vorliegenden Erfindung bedeutet "multidirektional", dass die Fasern nicht ausschließlich parallel zueinander in der Matrix angeordnet sind, sondern in vielen verschiedenen Richtungen verlaufen können. So kann es sowohl Fasern geben, die parallel zur Längsachse des Implantats verlaufen, als auch solche, die rechtwinklig oder schräg zu dieser Achse verlaufen, so dass praktisch alle denkbaren Faserorientierungen möglich sind. Die Fasern können hierbei auch einen gebogenen Verlauf aufweisen. Multidirektionale Fasern können insbesondere eine zufällige oder eine gezielte multidirektionale Anordnung aufweisen.

Eine "zufällig multidirektionale" Anordnung (auch als multidirektional-unorientierte Anordnung bezeichnet) der Fasern bedeutet, dass die Fasern innerhalb der Kunststoffmatrix nicht parallel zueinander verlaufen, sondern ein zufälliges Fasernetzwerk bilden und somit praktisch alle möglichen Orientierungen innerhalb der Matrix aufweisen. Dies ist beispielsweise das Resultat der Herstellung von faserverstärkten Kunststoffbauteilen mittels des CFM-Verfahrens.

Hierbei können bedingt durch das Herstellungsverfahren zwar Muster oder bestimmte bevorzugte Anordnungen der Fasern innerhalb des Bauteils bzw. Implantats entstehen. Diese sind aber trotzdem als eine zufällig multidirektionale Anordnung im Sinne der Erfindung einzustufen, da diese Anordnung sich nicht an den später auf das Implantat einwirkenden Kräften orientieren und diesen nicht gezielt entgegenwirken. Vielmehr sind diese Anordnungen eine zufällige Konsequenz des Herstellungsverfahrens.

Die zufällig multidirektionale Ausrichtung der Fasern innerhalb aller Bereiche des Implantats, einschließlich des Gewindes, erhöht die Zugfestigkeit des Materials bei Einwirkung von Kräften aus allen Richtungen, da hierbei immer auch Fasern vorliegen, die in Längsrichtung belastet werden und somit eine erhöhte Stabilität vermitteln. Entsprechend können erfindungsgemäße Implantate mit zufällig multidirektionaler Faserausrichtung den vielfältigen mechanischen Belastungen, denen sie ausgesetzt sind, besser und zuverlässiger standhalten als Implantate mit unidirektionaler Faserausrichtung.

Eine zufällig multidirektionale Faserorientierung ist beispielsweise vorteilhaft gegenüber einer unidirektionalen oder parallelen Faseranordnung im Falle eines Zahnimplantats mit Außengewinde. Wenn die Fasern unidirektional entlang der Längsachse des Zahnimplantats ausgerichtet sind, können sie beispielsweise im Bereich der Gewindeflanken des Außengewindes, das zur Verankerung des Zahnimplantats genutzt wird, keine Verstärkung gegenüber Zug- bzw. Druckkräften gewährleisten, die entlang der Längsachse des Implantats wirken. Wenn eine starke Zugbelastung auf ein entsprechendes in den Knochen eingebrachtes Zahnimplantat entlang der Längsachse wirkt, stellen die Gewindeflanken des Außengewindes die wahrscheinlichsten Bruchstellen eines entsprechenden Implantats dar, da die Fasern aufgrund Ihrer Ausrichtung keine Verstärkung darstellen. Dies wäre im Fall einer zufällig multidirektionalen Anordnung der Fasern anders, da die Fasern auch schräg oder senkrecht zur Längsachse des Implantats verlaufen, wodurch ein stabilisierender Effekt insbesondere im Bereich der Gewindeflanken erzielt wird.

Eine "gezielt multidirektionale" Anordnung (auch als multidirektional-orientierte Anordnung bezeichnet) der Fasern bedeutet, dass die Fasern innerhalb der Kunststoffmatrix des erfindungsgemäßen Implantats eine zuvor durchdachte und/oder berechnete multidirektionale Anordnung aufweisen. Die Fasern sind entsprechend so angeordnet bzw. orientiert, dass sie unter Berücksichtigung der auf das Implantat unter Benutzung einwirkenden mechanischen Belastungen eine optimale Stabilität gewährleisten können.

Durch die gezielt multidirektionale Faserorientierung eines erfindungsgemäßen Implantats ist gewährleistet, dass die Verstärkung der mechanischen Eigenschaften, insbesondere der Zugfestigkeit, die durch die Fasern erzielt wird, wirksam ist bei mechanischen Belastungen in praktisch allen Bereichen des Implantats. Dabei sind die Fasern so ausgerichtet, dass sie insbesondere Kräften, die das Implantat vertikal belasten, optimal entgegenwirken. Auch seitlich auftretende Kräfte werden so kompensiert. Zudem kann die Faserorientierung im Implantat an die lokalen Eigenschaften der knöchernen Umgebung des Implantats angepasst werden, wodurch eine optimale, praktisch physiologische Kraftübertragung auf den Knochen erreicht werden kann. Wie auch der Knochen selbst kann ein entsprechendes Implantat hierdurch eine gewisse Anisotropie erhalten.

Durch die multidirektionale Faseranordnung können lokale Anisotropien innerhalb des Implantats aus faserverstärktem PEEK hervorgerufen werden und Zugmoduli/Elastizitätsmodule zwischen ca. 100 GPa in Faserverlaufsrichtung und 3-4 GPa quer zum Faserverlauf erzeugt werden. Diese sind notwendig, um die lokal unterschiedlich auftretenden Belastungen zu kompensieren.

Eine bevorzugte Ausführungsform des erfindungsgemäßen Implantats ist dadurch gekennzeichnet, dass es einen mit gezielt multidirektional angeordneten Langfasern verstärkten thermoplastischen Kunststoff mit einem Elastizitätsmodul E von 10-70 GPa umfasst.

Die Bezeichnungen bzw. Begriffe "gezielt multidirektional angeordnet" und "multidirektional angeordnet" können im Sinne der Erfindung als Synonyme verwendet werden. Dies heißt, dass "multidirektional angeordnet" im Kontext der vorliegenden Erfindung die gleiche Bedeutung wie "gezielt multidirektional angeordnet" hat.

Es hat sich gezeigt, dass sich durch die gezielt multidirektionale Ausrichtung der Langfasern stress-shielding Effekte auf den angrenzenden Knochen vermeiden lassen. Die Belastung für den Knochen entspricht aufgrund der gezielten Faserausrichtung der Belastung, die unter physiologischen Bedingungen auf den Knochen wirkt. Hierdurch können pathologische Umbauprozesse im an das Implantat angrenzenden Knochen und anderen Geweben verhindert werden.

In einer bevorzugten Ausgestaltung des erfindungsgemäßen Implantats sind die Orientierung der Fasern, der Fasergehalt, die Art der Fasern und/oder der Faserdurchmesser innerhalb des Implantats bereichsweise unterschiedlich. Es ist bevorzugt, dass diese bereichsweisen Unterschiede auf einer gezielten Faseranordnung beruhen.

Es ist vorteilhaft, dass in dieser Ausführungsform der Erfindung insbesondere die Orientierung der Fasern, der Fasergehalt, die Faserzusammensetzung und/oder die Faserdurchmesser innerhalb des Implantats bereichsweise gezielt an die jeweilige mechanische Beanspruchung angepasst werden können.

Unter "Faserzusammensetzung" im Sinne der vorliegenden Erfindung ist insbesondere die Zusammensetzung der Fasern aus verschiedenen Sorten von Fasern, wie beispielsweise Kohlefasern, Glasfasern, Zirkonoxidfasern, Aluminiumoxidfasern, Siliziumcarbidfasern oder Mischungen aus diesen Fasern. Zudem bezieht sich der Begriff Faserzusammensetzung auf die Zusammensetzung von Faserbündeln aus einzelnen Faserfilamenten einer oder mehrerer Sorten von Fasern.

Beispielsweise können in Bereichen besonderer mechanischer Beanspruchung gezielt mehr Fasern eingearbeitet werden. Hierbei kann auch die Faserorientierung entlang der spezifischen mechanischen Beanspruchung des jeweiligen Bereichs ausgerichtet werden, so dass das Implantat den hier wirkenden Kräften besonders gut standhalten kann.

Beispielsweise können in einer bevorzugten Ausführungsform eines erfindungsgemäßen Implantats im Bereich eines Gewindes, beispielsweise in einem Außengewinde, das zur Verankerung des Implantats im Knochen genutzt wird, vermehrt Fasern eingearbeitet werden, die rechtwinklig zur Längsachse der Implantats verlaufen und somit bei Zugbelastung entlang der Schraubenlängsachse die Stabilität des Gewindes erhöhen. Weiterhin ist es möglich, im Bereich der Gewindeflanken vermehrt Fasern einzuarbeiten, die dem Verlauf der Schraubenwindung folgen, was zu einer Verstärkung der Gewindeflanken bei Zugbelastung entlang der Implantatlängsachse führen würde.

Überraschenderweise ist es möglich durch diese Art des gezielten Einsatzes von verstärkenden Fasern die mechanische Belastung für den an das Implantat angrenzenden Knochen zu vermindern. Beispielsweise können die Bereiche eines erfindungsgemäßen Implantats, die keine großen mechanischen Belastungen erfahren, da sie z.B. nicht essentiell für die Verankerung des Implantats im Knochen oder für die Befestigung von Implantataufbauten am Implantat sind, mit einer geringeren Steifigkeit versehen werden, indem an diesen Stellen wenige oder keine verstärkenden Fasern eingearbeitet werden. Hierdurch würde die Kraftübertragung auf den Knochen an diesen Stellen vermindert, da das angrenzende Kunststoffmaterial des Implantats eine geringere Steifigkeit hat und sich unter Krafteinwirkung leichter verformen lässt, wodurch ein Teil der einwirkenden Kraft absorbiert wird und die resultierende Kraftübertragung auf den Knochen der physiologischen Belastung ähnelt.

Entsprechend ist es überraschenderweise möglich durch die gezielte Anordnung verstärkender Fasern in den verschiedenen an den Knochen angrenzenden Bereichen des Zahnimplantats mechanische Belastungen für den Knochen zu erzeugen, die denen, die durch eine natürliche Zahnaufhängung im Kieferknochen verursacht werden, sehr ähnlich sind. Eine solche an die knöcherne Umgebung des Implantats und an die spezifischen Belastungen, die auf das Implantat einwirken, angepasst Gestaltung des erfindungsgemäßen Implantats erzeugt daher eine fast physiologische Belastung für den angrenzenden Knochen und ist somit gegenüber bekannten Implantaten äußerst vorteilhaft.

Die Anordnung der Fasern im Implantat muss hierfür an die Art des Implantats, den Implantationsort und die dort auf das Implantat wirkende Kraft angepasst werden. Bei Zahnimplantaten ist hierbei unter anderem wichtig, welcher Zahn mit Hilfe des Implantats ersetzt werden soll, und wie das Implantat nach dem Einsetzen, beispielsweise beim Kauen, belastet wird.

Dem Fachmann ist bekannt, wie er solche Belastungsdaten für den jeweiligen Implantattyp erhalten kann. Hierfür können beispielsweise bildgebende Verfahren genutzt werden, die Informationen über die räumlichen Strukturen sowie die Knochendichte der Umgebung, in die das Implantat eingesetzt werden soll, generieren. Insbesondere können hierfür ein Digitaler Volumentomograph (DVT) oder ein Computertomograph (CT) genutzt werden, die einen DICOM (Digital Imaging and Communications in Medicine) Datensatz bzgl. der dreidimensionalen Umgebung des Implantats generieren. Zudem können z.B. für Zahnimplantate relevante Patientenparameter wie Kaukraft bestimmt und elektronisch erfasst werden. Diese Informationen kann der Fachmann beispielsweise mit Hilfe eines Computerprogramms so verarbeiten, dass die optimale Faserverteilung im erfindungsgemäßen Implantat berechnet wird. Mit Hilfe dieser Informationen kann der Fachmann ein erfindungsgemäßes Implantat aus faserverstärktem thermoplastischem Kunststoff herstellen, das eine bereichsweise unterschiedliche Faserverteilung aufweist und an die individuellen Anforderungen in Bezug auf die einwirkende Belastung und die knöcherne Umgebung angepasst ist.

Weiterhin ist ein Implantat aus faserverstärktem Kunststoff, das gezielt multidirektional angeordneten Langfasern enthält, von einem durchschnittlichen Fachmann eindeutig von einem Implantat, das zufällig multidirektional angeordnete Langfasern enthält, zu unterscheiden.

Bei der gezielten Anordnung der Fasern im Implantat entsteht eine spezifische Anordnung der Fasern in bestimmten Regionen des Implantats, die zu einem charakteristischen Muster der gezielt angeordneten Fasern innerhalb des Implantats führt. Hierbei werden die Fasern spezifisch an den im Implantat nach dem Einsetzen in den Knochen auftretenden Belastungslinien/-richtungen ausgerichtet. Diese Anordnung unterscheidet sich von einer zufälligen Anordnung, wie sie beispielsweise bei der Herstellung des Implantats durch ein Spritzguss- oder CFM-Verfahren entsteht.

Es ist in einer routinemäßigen mikroskopischen Analyse oder einer ähnlichen Analyse, bei der die Anordnung der Fasern innerhalb des Implantats sichtbar gemacht werden kann, eindeutig erkennbar, ob die Fasern innerhalb des Implantats gezielt oder zufällig angeordnet sind. Im Falle der gezielten Anordnung kann beispielsweise in bestimmten Regionen des Implantats, wie dem apikalen Ende, eine sehr spezifische Faseranordnung vorgefunden werden, die sich von der Faseranordnung in anderen Regionen des Implantats unterscheidet. Am apikalen Ende ist das Implantat völlig anderen mechanischen Belastungen ausgesetzt als am koronalen Ende oder in der Mitte des Implantats. Dies spiegelt sich entsprechend in der gezielten Faseranordnung wider. Selbiges gilt für die äußeren Schichten im Vergleich zu zentralen Schichten.

Daher kann bei einer gezielten Faseranordnung, wie im Rahmen der vorliegenden Erfindung beansprucht, in den unterschiedlichen Regionen des Implantats eine unterschiedliche, Regionen-spezifische charakteristische Faseranordnung vorgefunden werden. Diese kann weiterhin von der späteren Position des Implantats im Kiefer abhängen. Insbesondere kann es Faserbündel geben, die entlang der Regionen-spezifischen Belastungslinien verlaufen.

Dies ist bei einer zufälligen Faseranordnung nicht der Fall. Hier ordnen sich die Fasern stattdessen zufällig an, unabhängig von den später auf das Implantat einwirkenden Belastungen. Dabei können zwar auch Muster oder charakteristische Strukturen entstehen. Diese sind aber nur dem Herstellungsverfahren geschuldet und spiegeln nicht die Belastungslinien innerhalb des Implantats nach Einsetzen in den Knochen wider.

So kann es bei der Herstellung von entsprechenden Implantaten mit zufällig multidirektionaler Ausrichtung, z.B. mittels des CFM-Verfahrens, zu einer Ausrichtung von Fasern entlang der äußeren Windungskonturen der Schraube kommen. Diese Ausrichtung ist aber als zufällig einzuordnen, da sie z.B. unabhängig davon entsteht, ob sich die jeweilige Windung am apikalen oder koronalen Ende des Implantats befindet. Die Ausrichtung orientiert sich also nicht an den tatsächlich auf das Implantat einwirkenden Kräften, sondern resultiert rein aus dem Herstellungsverfahren.

Da bestimmte Herstellungsverfahren auch mit bestimmten Faseranordnungsmustern assoziiert sind, können diese eindeutig von einer gezielten Anordnung, bei der die Fasern entlang der charakteristischen Belastungslinien eines eingesetzten Implantats verlaufen, unterschieden werden. Aufgrund der Tatsache, dass auch bei Herstellungsverfahren, die zu einer zufälligen Faseranordnung führen, Fasermuster entstehen können, die für das jeweilige Herstellungsverfahren charakteristisch sind, kann es auch nicht durch einen sehr unwahrscheinlichen Zufall dazu kommen, dass ein Implantat, das durch ein Verfahren hergestellt wurde, bei dem es zu einer zufälligen Faseranordnung kommt, die gleiche Faseranordnung aufweist, wie ein Implantat, das eine gezielte Faseranordnung aufweist.

Daher sind Implantate mit einer gezielten Langfaserausrichtung für einen Fachmann immer von Implantaten mit zufälliger Faserausrichtung zu unterscheiden.

In einer weiteren Ausführungsform des erfindungsgemäßen Implantats beträgt der Elastizitätsmodul E 20-60 GPa. Es hat sich gezeigt, dass erfindungsgemäße Implantate, insbesondere Zahnimplantate, die Elastizitätsmodule im Bereich von 20-60 GPa aufweisen, besonders vorteilhaft sind, da sich überraschenderweise gezeigt hat, dass solche Implantate eine längere Nutzungsdauer im Vergleich zu Implantaten mit E-Modulen über 60 GPa oder unter 20 GPa haben.

In einer weiteren Ausführungsform des erfindungsgemäßen Implantats beträgt der Elastizitätsmodul E 30-50 GPa. Entsprechende Implantate sind für den Träger des Implantats besonders komfortabel sind, da der Unterschied zu der natürlichen/gesunden Situation ohne Implantat subjektiv nicht feststellbar ist.

In einer weiteren Ausführungsform des erfindungsgemäßen Implantats beträgt der Elastizitätsmodul E 35-45 GPa. Für Implantate mit einem E-Modul im Bereich von 35-45 GPa zeigte sich, dass diese besonders vorteilhafte mechanische Eigenschaften aufweisen, so dass es auch lange Zeit nach Einsetzten des Implantat praktisch zu keinen wesentlichen Veränderung des angrenzenden Knochens kommt. Dies war insbesondere überraschend für Zahnimplantate, da Zahnschmelz, der die natürliche äußere Schicht von Zähnen darstellt, mit 50-85 GPa einem höheren E-Modul aufweist als diese Ausführungsform des erfindungsgemäßen Implantats.

In einer weiteren bevorzugten Ausführungsform ist das Implantat dadurch gekennzeichnet, dass der thermoplastische Kunststoff ausgewählt ist aus einer Gruppe umfassend PEEK, PA, PE, POM, PMMA, PVAc, PU, und/oder PAEK oder Polymerblends derselben oder auf Basis derselben.

In einer weiteren bevorzugten Ausführungsform ist das Implantat dadurch gekennzeichnet, dass der thermoplastische Kunststoff PEEK ist.

Bei den Polymerblends kann es sich um Blends aus zwei oder mehreren Kunststoffen, insbesondere aus zwei oder mehreren biokompatiblen Kunststoffen, vorzugsweise aus zwei oder mehreren biokompatiblen thermoplastischen Hochleistungskunststoffen handeln.

Solche Polymerblends können beispielsweise aus den Kunststoffen ausgewählt aus der Gruppe umfassend PEEK, PA, PE, POM, PMMA, PVAc, TPU, PAEK oder aus Polymerblends bzw. Kunststoffblends eines oder mehrerer dieser Kunststoffe mit einem oder mehreren anderen Polymeren bzw. Kunststoffen, insbesondere anderen biokompatiblen Polymeren bzw. Kunststoffen, gebildet sein.

Polyetherketone (kurz PEK) sind Polymere, in deren molekularen Rückgrat abwechselnd Keton- und Etherfunktionalitäten vorkommen. Am gebräuchlichsten sind Polyaryletherketone (PAEK), bei denen sich zwischen den funktionellen Gruppen jeweils eine in (1,4)-Position verknüpfte Arylgruppe befindet. Das sehr starre Rückgrat verleiht den Materialien im Vergleich zu anderen Kunststoffen sehr hohe Glasübergangs- und Schmelztemperaturen. Der bei weitem verbreitetste dieser hochtemperaturfesten Werkstoffe ist das Polyetheretherketon (PEEK).

PEEK ist ein hochtemperaturbeständiger thermoplastischer Kunststoff und gehört zur Stoffgruppe der Polyaryletherketone. PAEK und PEEK sind gegenüber fast allen organischen und anorganischen Chemikalien und bis etwa 280 °C auch gegen Hydrolyse beständig. Im festen Zustand kann PEEK mit einer Fräse bearbeitet, gedreht oder gebohrt werden. PEEK schmilzt bei einer, im Vergleich zu den meisten anderen Thermoplasten, sehr hohen Temperatur von 335 °C und kann im flüssigen Zustand im Spritzgussverfahren, 3D-Druckverfahren, CFM-Verfahren, Formpresseverfahren oder per Extruder geformt werden. Aus Pulvern von PEEK ist durch Einsatz von Lasern im SLS Verfahren die Herstellung von 3D-Formstücken möglich. Dies macht PEEK zu einem besonders geeigneten Material für die Herstellung von Implantaten, da es in verschiedenen Verfahren eingesetzt werden kann, die zur Herstellung von erfindungsgemäßen Implantaten eingesetzt werden können und gleichzeitig unter physiologischen und auch pathologischen Bedingungen, wie sie im Körper, wo die Implantate eingesetzt werden, vorkommen, fest und widerstandsfähig ist.

Die Verwendung von PEEK als Werkstoff für die Fertigung von Implantaten ist besonders vorteilhaft aufgrund seiner hohen Festigkeit und Steifigkeit. Aufgrund seines ElastizitätsModuls von 3-5 GPa wurde erfindungsgemäß gefunden, dass es sich besonders gut für die Herstellung von Implantaten eignet, da sich insbesondere durch Zusatz von verstärkenden Fasern und ggf. anderen Zusätzen für Implantate besonders vorteilhafte mechanische Eigenschaften des Materials erzielen lassen, die auf die knöcherne Umgebung des Implantats abgestimmt sind. Aufgrund der guten mechanischen Grundvoraussetzungen, die PEEK mit sich bringt, ist eine Anpassung der mechanischen Eigenschaften durch Compoundzusätze und Fasern besonders vereinfacht und bereits geringfügige Zusätze ermöglichen einen bedeutenden Effekt. Zudem hat PEEK eine geringe Dichte (1,32 g/cm3), was sehr vorteilhaft für die Herstellung von erfindungsgemäßen Implantaten ist, da die zusätzliche Belastung für das umgebende Gewebe und für den Patienten, der das Implantat in sich trägt, minimiert wird.

Aufgrund seiner guten Chemikalienbeständigkeit und Stabilität gegenüber Lösungsmitteln ist PEEK besonders geeignet für die Herstellung von Implantaten. Beim Einbringprozess kommen Implantate, insbesondere Zahnimplantate, häufig mit Chemikalien und Lösungsmitteln in Kontakt, beispielsweise wenn es zu Verklebung mit prothetischen Komponenten kommt, die auf dem Implantat aufgebaut werden. Hierbei hat ein Implantat aus PEEK den entscheidenden Vorteil, dass es seine Form und Festigkeit und andere Materialeigenschaften unter dem Einfluss der hierzu verwendeten Klebstoffe, die verschiedene Chemikalien und Lösungsmittel enthalten, nicht verändert. Auch für Implantate, an Körperoberflächen oder zugänglichen Körperbereichen wie dem Mundraum mit der Außenwelt in Kontakt kommen, ist diese Beständigkeit des Materials von Vorteil.

Ein weiterer Vorteil von erfindungsgemäßen Implantaten aus PEEK ist, dass sie eine hohe Strahlungsfestigkeit aufweisen, insbesondere gegenüber UV-Strahlen. Beim Verkleben von Implantaten mit prothetischen Aufbaukomponenten wird häufig UV-Strahlung eingesetzt, um den verwendeten Klebstoff zu härten. Hierbei wird auch das Implantat der Strahlung ausgesetzt. PEEK ist daher besonders geeignet für die Herstellung von Implantaten, da andere Kunststoff unter dem Einfluss von Strahlung und insbesondere UV-Strahlen brüchig und porös werden.

Erfindungsgemäße Implantate aus PEEK haben insbesondere gegenüber Implantaten aus Metall den Vorteil der Röntgentransparenz, was sich bei der Röntgen-Untersuchung und anderen bildgebenden Verfahren, die an Implantatträgern durchgeführt werden, sehr vorteilhaft ist, da die Untersuchung des umgebenden Gewebes nicht beeinträchtigt wird. Entsprechend müssen erfindungsgemäße Implantate bei solchen Untersuchung nicht umständlich entfernt werden, wie dies beispielsweise bei Implantaten aus Metall der Fall ist.

Erfindungsgemäße Implantate aus PEEK haben zudem im Vergleich zu bekannten Kunststoffimplantaten den Vorteil der Biokompatibilität, die durch die Verwendung von Compoundzusätzen, insbesondere im Oberflächenbereich eines solchen Implantats, sogar noch erhöht werden kann. Zudem sind Implantate aus PEEK hochbeständig gegenüber Verscheiß durch Abrieb, was insbesondere bei der Verwendung für zahnmedizinische Implantate von großem Vorteil ist, da diese aufgrund der im Mundraum wirkenden Kaukräfte besonders starken Reibungskräften ausgesetzt sind. Erfindungsgemäße Implantate aus PEEK sind zudem faserverstärkt, was sie besonders widerstandsfähig gegen diese Kräfte macht. Im Gegensatz dazu sind bekannt Kunststoffimplantate diesen im Mundraum wirkenden Kräften nicht gewachsen und bilden daher keine gleichwertige funktionelle Alternative zu den erfindungsgemäßen Implantaten, insbesondere nicht als für den dauerhaften Zahnersatz eingesetzte Implantate.

Zudem zeigen Implantate aus PEEK eine äußerst geringe Plaqueadhärenz, was durch den Zusatz von geeigneten Compoundzusätzen noch verstärkt werden kann. Dies ist insbesondere für den Einsatz in der Zahnmedizin von großem Vorteil, da Plaque (Zahnbelag), der u.a. aus Mikroorganismen besteht, sich insbesondere auf der Oberfläche von Zahnimplantaten absetzt, was beispielsweise zu Entzündungen des umliegenden Gewebes, Zahnkaries, Parodontitis und Gingivitis führen kann. Bekannte Kunststoffimplantate mit einer im Vergleich zu erfindungsgemäßen Implantaten erhöhten Elastizität sind besonders anfällig für Plaqueablagerungen, da sie schnell porös werden, was die Plaqueablagerung zusätzlich begünstigt. Überraschenderweise zeigt sich, das die Gefahr der Plaqueablagerung auf der Oberfläche von erfindungsgemäßen Implantaten aus PEEK minimiert werden konnte aufgrund des Zusammenwirkens der Plaque-abweisenden Eigenschaften von PEEK, die durch Compoundzusätze noch verstärkt werden kann, und der mechanischen Eigenschaften der erfindungsgemäßen Implantate, die eine Porenbildung auf der Implantatoberfläche.

Erfindungsgemäße Implantate aus PEEK haben zudem der Vorteil, dass sie eine geringe Wärmeleitfähigkeit haben, was insbesondere bei Zahnimplantaten positiv ist, da hier die Hitze, der die Implantate beispielsweise durch Kontakt zu heißen Speisen im Mundraum ausgesetzt sind, nicht direkt auf den Knochen weitergeleitet werden, was zu Schmerzen beim Implantatträger führen kann. Dies ist insbesondere bei bekannten Implantatlösungen aus Metall ein Problem.

Zudem weisen Implantate aus PEEK praktisch eine geringe elektrische Leitfähigkeit auf, wodurch das Risiko des Implantatträgers, im Falle des Kontakts mit einer starken Stromquelle schwere gesundheitliche Schäden davonzutragen, verringert wird insbesondere im Vergleich zu metallischen Implantaten.

Polyamide (PA) sind lineare Polymere mit sich regelmäßig wiederholenden Amidbindungen entlang der Hauptkette. Die Amidgruppe kann als Kondensationsprodukt einer Carbonsäure und eines Amins aufgefasst werden. Die dabei entstehende Bindung ist eine Amidbindung, die hydrolytisch wieder spaltbar ist. Polyamide werden wegen ihrer hervorragenden Festigkeit und Zähigkeit oft als Konstruktionswerkstoffe verwendet. PA zeigen eine gute chemische Beständigkeit gegenüber organischen Lösungsmitteln, sind kältefest, stoßbelastbar, schlagzäh, abriebfest selbst bei rauem Gleitpartner, und besitzen ein hohes Arbeitsvermögen.

Polyethylene (PE) sind wachsartig weich und haben eine antiadhäsive Oberfläche. Polyethylene sind reinster Kohlenwasserstoff. Ebenso wie reiner Kerzenwachs bestehen sie aus Kohlenwasserstoffketten, nur dass die Molekülketten beim Polyethylen länger sind. Polyethylene haben eine niedrige Dichte, gute Zähigkeit und sehr gute Chemikalienfestigkeit.

Polyoxymethylen (POM) ist ein hochmolekularer thermoplastischer Kunststoff und ist ein universeller Kunststoff, der häufig für Funktionsteile in der Feinmechanik und im Apparatebau genutzt wird. Der Konstruktionswerkstoff bietet hervorragende Eigenschaften, so z.B. niedrigen Reibwiderstand, gute Abriebfestigkeit, hervorragendes Federvermögen, hohe Ermüdungsfestigkeit bei wechselnder Beanspruchung, gute elektrische Eigenschaften, hohe Durchschlagfestigkeit, einen niedrigen dielektrischen Verlustfaktor, gute Chemikalienfestigkeit - besonders gegen Lösungsmittel - und er ist sehr beständig gegen Spannungsrissbildung.

Polymethylmethacrylat (PMMA) ist ein synthetischer, transparenter, thermoplastischer Kunststoff. PMMA ist kratzfest und von hoher optischer Qualität. Es ist ein hochwertiger klarsichtiger Kunststoff mit einer sehr hohen Steifigkeit und guter Witterungsbeständigkeit.

Polyvinylacetat (PVAc, manchmal auch nur PVA) ist ein thermoplastischer Kunststoff aus der Gruppe der Polyvinylester, der mittels radikalischer Polymerisation synthetisiert wird.

Thermoplastisches Polyurethan (TPU) ist ein elastischer Werkstoff mit einer sehr guten Verschleißfestigkeit, gepaart mit einem hohen Rückstellvermögen.

Vorzugsweise enthält der faserverstärkte thermoplastische Kunststoff Kohlefasern, Glasfasern, Zirkonoxidfasern, Aluminiumoxidfasern, Siliziumcarbidfasern oder Mischungen aus diesen Fasern.

Kohlefasern im Sinne der vorliegenden Erfindung sind industriell hergestellte Fasern aus kohlenstoffhaltigen Ausgangsmaterialien, die durch an den Rohstoff angepasste chemische Reaktionen in graphitartig angeordneten Kohlenstoff umgewandelt werden. Man unterscheidet isotrope und anisotrope Typen: Isotrope Fasern besitzen nur geringe Festigkeiten und geringere technische Bedeutung, anisotrope Fasern zeigen hohe Festigkeiten und Steifigkeiten bei gleichzeitig geringer Bruchdehnung in axialer Richtung.

Es werden, ohne Limitierung, folgende Kohlefaser unterschieden, die im Rahmen der vorliegenden Erfindung verwendet werden können: HT (hochfest / High Tensity / High Tenacity), UHT (sehr hochfest / Ultrahigh Tenacity), LM (Low Modulus), IM (Intermediate Modulus), HM (hochsteif / High Modulus), UM (Ultra Modulus), UHM (Ultra High Modulus), UMS (Ultra Modulus Strength), HMS (hochsteif / hochfest / High Modulus / High Strength).

Üblicherweise werden 1.000 bis 24.000 Filamente zu einem Multifilamentgarn (Roving) zusammengefasst, das aufgespult wird. Die Weiterverarbeitung zu textilen Halbzeugen wie z. B. Geweben, Geflechten oder Multiaxialgelegen erfolgt auf Webmaschinen, Flechtmaschinen oder Multiaxial-Wirkmaschinen bzw. im Bereich der Herstellung von faserverstärkten Kunststoffen direkt auf Pre-Preg-Anlagen, Strangziehanlagen (Pultrusionsanlagen) oder Wickelmaschinen.

Die Verwendung von Kohlefasern zur Faserverstärkung des thermoplastischen Kunststoffs ist für die vorliegende Erfindung von großem Vorteil, da Kohlefasern eine besonders hohe Zugfestigkeit von 3,5-4,5 GPa und einen hohen Zug-E-Modul von 230-395 GPa ausweisen, je nach Kohlefasertyp. Diese mechanischen Eigenschaften ermöglichen es, durch die Verwendung von Kohlefasern zur Faserverstärkung eines erfindungsgemäßen Implantats dem thermoplastischem Kunststoff, der für sich genommen, den spezifischen mechanischen Anforderungen an ein Implantat nicht gewachsen wäre, die nötige Stabilität zu verleihen.

Aufgrund dieser spezifischen mechanischen Eigenschaften sind Kohlefasern besonders geeignet, um die mechanischen Eigenschaften des erfindungsgemäßen Implantats auf die im Körper auf das Implantat wirkenden Kräfte abzustimmen, so dass die optimale Balance zwischen Steifigkeit und Elastizität eingestellt werden kann. Insbesondere die außerordentliche Zugfestigkeit von 3,5-4,5 GPa ermöglicht es, dass bereits bei Verwendung eines relativ geringen Anteils von Kohlefasern im Implantat die Zugfestigkeit und Stabilität des verwendeten thermoplastischen Kunststoffs entscheidend verbessert wird. Auch die Bruchdehnung von 1,1-1,5 % ist von Vorteil bei der Verwendung im Rahmen der vorliegenden Erfindung, da hierdurch beim Einwirken besonders starker Kräfte auf das erfindungsgemäße Implantat eine gewisse Elastizität sichergestellt wird, ohne dass dabei die Stabilität beeinträchtigt wird.

Ein wichtiger Vorteil der Verwendung von Kohlefasern im Rahmen der vorliegenden Erfindung ist zudem, dass sich eine besonders stabile Verbindung zwischen den verstärkenden Kohlefasern und der umgebenden Kunststoffmatrix herstellen lässt. Hierdurch wird auch unter hoher mechanischer Belastung des Implantats verhindert, dass sich der Kunststoff und die verstärkenden Fasern gegeneinander verschieben. Durch diese besonders feste Verbindung zwischen thermoplastischer Kunststoffmatrix und Kohlefasern werden die mechanischen Eigenschaften der Kohlefasern besonders wirksam auf das erfindungsgemäße Implantat übertragen.

Im Sinne der Erfindung ist eine Glasfaser eine aus Glas bestehende lange dünne Faser. Bei der Herstellung werden aus einer Glasschmelze dünne Fäden gezogen. Glasfasern haben eine Zugfestigkeit von 1,8-5 GPa, einen Zug-E-Modul von 70-90 GPa und eine Bruchdehnung von 5%. Aufgrund dieser mechanischen und physikalischen Eigenschaften sind Glasfasern besonders gut zur Verarbeitung als Faserverstärkung von erfindungsgemäßen Implantaten geeignet. Insbesondere durch die hohe Bruchdehnung von 5 % wird eine hohe Elastizität von mit Glasfasern verstärkten erfindungsgemäßen Implantaten ermöglicht. Dies ist bei außergewöhnlich hoher mechanischer Belastung von Vorteil, da das Implantat unter solchen Bedingungen seine Form anpassen kann, ohne dass es zum Bruch der verstärkenden Fasern kommt. Hierdurch wird auch die Kraftübertragung auf den angrenzenden Knochen vermindert, so dass ein pathologischer Knochenumbau verhindert wird. Gleichzeitig wird dem Implantat aufgrund der hohen Zugfestigkeit die nötige Stabilität verliehen, um den Anforderungen nach dem Einbau in den Körper gewachsen zu sein.

Ein weiterer wichtiger Vorteil der Verwendung von Glasfasern in erfindungsgemäßen Implantaten ist, dass sie alterungs- und witterungsbeständig sind, so dass sie über mehrere Jahre hinweg und somit praktisch lebenslang im Körper verbleiben können, ohne dass sie durch die feuchte Umgebung, beispielsweise im Mundraum im Fall von Zahnimplantaten, ihre vorteilhaften mechanischen Eigenschaften einbüßen würden. Zudem sind sie chemisch resistent, so dass auch der Einsatz von chemischen Klebstoffen zur Befestigung von Implantataufbauten keine Beeinträchtigung oder Beschädigung eines mit Glasfasern verstärkten Implantats hervorruft.

Zudem hat die Verwendung von Glasfasern insbesondere für die Herstellung von erfindungsgemäßen Implantaten, die von außen sichtbar sind, wie beispielsweise Zahnimplantate, den wichtigen Vorteil, dass sie das optische Erscheinungsbild eines Kunststoffes nicht signifikant verändern, so dass die Herstellung von erfindungsgemäßen Implantaten in einer Farbe, die Zähnen oder Knochen ähnelt, im Falle einer Faserverstärkung durch Glasfaserns erleichtert ist.

Im Sinne der vorliegenden Erfindung handelt es sich bei Zirkonoxidfasern und Aluminiumoxidfasern um keramischen Fasern aus anorganischem, nicht-metallischem Material, die zu den oxidischen Keramikfasern zählen. Aufgrund ihrer sehr hohen Zugfestigkeit von 1,7-2,9 GPa und des sehr variablen und mitunter außerordentlich hohen Zug-E-Modul von 15-370 GPa sind diese Fasern besonders vorteilhaft als Verstärkungsfasern für erfindungsgemäße Implantate, da die mechanischen Eigenschaften der Implantate sich sehr variabel auf die jeweiligen zu erwartenden mechanischen Belastungen im Körper anpassen lassen. Auch bei Verwendung eines relativ geringen Faseranteils lassen sich somit sehr belastbare Implantate herstellen. Aufgrund Ihrer Bruchdehnung von 0,6-1,1% haben Zirkonoxidfasern und Aluminiumoxidfasern zudem beim Einsatz in erfindungsgemäßen Implantaten den Vorteil, dass diese auch unter sehr hohen mechanischen Belastungen eine gewisse Elastizität aufweisen, die ein Verformung des Implantats ermöglicht, bevor es zum Bruch kommt. Hierdurch wird auch die Kraftübertragung auf den angrenzenden Konchen vermindert, so dass ein pathologischer Knochenumbau verhindert wird. Zirkonoxidfasern und Aluminiumoxidfasern haben zudem aufgrund ihrer hellen Eigenfarbe insbesondere bei der Verwendung in der Zahnmedizin einen optischen Vorteil, da sich durch die Verwendung dieser Fasern für die Verstärkung erfindungsgemäße Implantate in zahnähnlicher Farbe leichter herstellen lassen.

Siliziumcarbidfasern im Sinne der vorliegenden Erfindung (auch als SIC-Fasern oder Nicalon bezeichnet) werden ausgehend von Dichlordimethylsilan hergestellt. Dieses polymerisiert zu Polydimethylsilan, welches beim Erhitzen unter Kondensation und Abspaltung von Chlor zu Polycarbosilan umlagert. Daraus werden Fasern gezogen, die später in Siliciumoxycarbidfasern pyrolysiert werden.

Siliciumoxycarbidfasern (SIC-Fasern) können im Rahmen der vorliegenden Erfindung zur Fertigung von SIC-Faser-verstärkten Implantaten aus thermoplastischem Kunststoff verwendet werden. Durch diese Faserverstärkung wird entsprechenden Implantaten eine sehr hohe Bruchzähigkeit, die im Bereich von Metallen wie zum Beispiel Grauguss liegt, verliehen. Dies wird insbesondere durch die hohe Zugfestigkeit von 2,6-3,4 GPa bei einen Zug-E-Modul von 170-420 GPa der SIC-Fasern ermöglicht. Zudem weisen Siliciumoxycarbidfasern eine Bruchdehnung von 0,6-1,9% auf, wodurch unter sehr hohen mechanischen Belastungen eine gewisse Elastizität gewährleistet wird, die eine Verformung des Implantats ermöglicht, bevor es zum Bruch kommt. Hierdurch wird auch die Kraftübertragung auf den angrenzenden Konchen vermindert, so dass ein pathologischer Knochenumbau verhindert wird.

Ein wichtiger Vorteil der Verwendung von SIC-Fasern ist, dass hierdurch nicht nur die Bruchzähigkeit erfindungsgemäßer Implantate erhöht wird, sondern auch vorteilhafte optische Eigenschaften, insbesondere eine Erhöhung der Transluzenz (Lichtdurchlässigkeit), ermöglicht wird, was insbesondere bei der Verwendung in zahnmedizinischen Implantaten vorteilhaft ist.

In einer bevorzugten Ausführungsform des erfindungsgemäßen Implantats haben die verstärkenden Fasern einen Durchmesser von 0,1 bis 100 µm, bevorzugt 4 bis 10 µm.

Als Durchmesser einer Faser im Sinne der Erfindung wird ihre Ausbreitung entlang der zur Axialrichtung senkrecht verlaufenden Richtung bezeichnet.

Im Rahmen der vorliegenden Erfindung können die verstärkenden Fasern unterschiedliche Durchmesser aufweisen, wobei ein erhöhter Durchmesser mit einer höheren Beständigkeit gegenüber Verschleiß und einer erhöhten Stabilität gegenüber Zugbelastungen einhergeht. Die Fasern können aus verschiedenen Arten von Faserfilamenten zusammengesetzt sein und auch Nanopartikel, die mit den Fasern verschmelzen, einschließen. Die Fasern können dabei einen Durchmesser von 0,1 µm bis zu 100 µm haben. Die Bruchzähigkeit des resultierenden Verbundmaterials, aus dem das erfindungsgemäße Implantat hergestellt ist, kann durch die spezifische Zusammensetzung der Fasern so beträchtlich erhöht und spezifisch eingestellt werden.

Eine bevorzugte Ausführungsform der Erfindung betrifft ein Implantat aus Kunststoff, dadurch gekennzeichnet, dass es einen mit multidirektional angeordneten Langfasern verstärkten thermoplastischen Kunststoff mit einem Elastizitätsmodul E von 30-50 GPa umfasst, wobei die verstärkenden Fasern einen Durchmesser von 4 bis 10 µm haben.

In einer weiteren bevorzugten Ausführungsform besteht das erfindungsgemäße Implantat aus einem mit multidirektional angeordneten Langfasern verstärkten thermoplastischen Kunststoff mit einem Elastizitätsmodul E von 30-50 GPa, wobei die verstärkenden Fasern einen Durchmesser von 4 bis 10 µm haben.

Es hat sich gezeigt, dass erfindungsgemäße Implantate, in denen Fasern mit einem Durchmesser im Bereich von 4 bis 10 µm verarbeitet wurden, besonders vorteilhaft sind, da sich solche Fasern gut verarbeiten lassen und auch in sehr feinen Strukturen der erfindungsgemäßen Implantate, wie beispielsweise in Gewindeflanken, angeordnet werden können. Dies ist bei Fasern mit größeren Durchmessern nicht ohne weiteres möglich. Ein wichtiger Vorteil von erfindungsgemäßen Implantaten, die verstärkende Fasern mit einem Durchmesser von 4 bis 10 µm haben, gegenüber Implantaten, die verstärkende Fasern mit einem geringeren Durchmesser haben, ist, dass das Herstellungsverfahren deutlich weniger aufwendig ist, da die Anzahl der einzuarbeitenden Fasern deutlich geringer ist, wenn der prozentual gleiche Faseranteil und somit ähnliche verstärkende Eigenschaften erzielt werden sollen.

Überraschenderweise hat sich gezeigt, das Implantate, die sowohl ein Elastizitätsmodul E im Bereich von 30-50 GPa als auch Langfasern mit einem Durchmesser von 4-10 µm aufweisen, besonders stabil und widerstandfähig nach dem Einsetzen in den Knochen sind. Gleichzeitig war es völlig überraschend, das bei solchen Implantaten die Belastungen für den Knochen über sehr lange Zeiträume besonders niedrig waren, auch im Vergleich zu anderen hier offenbarten Implantaten, die nur eines dieser Merkmale aufweisen. Dies führt dazu, dass entsprechende Implantate eine besonders lange Haltbarkeit nach dem Einsetzen in den Knochen haben.

Diese besonderen Effekte der guten Verträglichkeit für den Knochen und gleichzeitigen Widerstandfähigkeit traten nur bei dieser spezifischen Kombination von Merkmalen aus einem Elastizitätsmodul von 30-50 GPa und Fasern mit einem Durchmesser von 4-10 µm auf. Bei einer Erhöhung oder Verminderung des Elastizitätsmoduls unter Verwendung von 4-10 µm dicken Fasern traten negative stress-shielding Effekte an den angrenzenden Knochen auf. Wenn dünnere Fasern verwendet wurden, kam es bei dauerhafter Belastung zu einem Bruch der Fasern, der viel früher auftrat als bei Verwendung von 4-10 µm dicken Fasern. Der Faserbruch ging mit einer kontinuierlichen Abnahme des Elastizitätsmoduls einher und führte auch zu Schäden an den angrenzenden Knochen.

Bei der Verwendung von dickeren Fasern mit einem Durchmesser von über 10 µm wurde festgestellt, dass es unter dauerhafter Belastung der Implantate im Mundraum, wo sie auch den Speichelenzymen ausgesetzt sind, überraschend zu einer Versteifung des Implantats und zu einer sukzessiven Zunahme des Elastizitätsmoduls kam, was sich sehr negativ auf die angrenzenden Knochen auswirkt. Nur bei der Verwendung von Fasern mit einem Durchmesser von 4-10 µm konnte das vorteilhafte Elastizitätsmodul von 30-50 GPa über lange Zeiträume unter Belastung im Mundraum stabil gehalten werden, so dass auch sehr lange nach dem Einsetzen des Implantats die erfindungsgemäßen Vorteile aufrecht erhalten werden konnten. Dies ist auf einen überraschenden und unvorhergesehenen synergistischen Effekt des spezifischen Faserdurchmessers von 4-10 µm und des spezifischen Bereichs des Elastizitätsmoduls von 30-50 GPa zurückzuführen.

In einer Ausführungsform zeichnet sich das erfindungsgemäße Implantat dadurch aus, dass der Fasergehalt des Kunststoffs 10-80 Vol.-%, vorzugsweise 40-60 Vol.-% ist.

Als Fasergehalt im Sinne der vorliegenden Erfindung wird der Volumenanteil des faserverstärkten Kunststoffes, der von Fasern ausgefüllt wird, bezeichnet. Der Fasergehalt wird in der Einheit Volumen-% (Vol.-%) angegeben. Über den Fasergehalt lassen sich die spezifischen mechanischen Eigenschaften des erfindungsgemäßen Implantats einstellen. Ein höherer Fasergehalt geht hierbei mit einer erhöhten Stabilität und Bruchzähigkeit einher.

Es ist vorteilhaft, dass Implantate mit stark variierendem Fasergehalt von 10-80 Vol.-% geeignete mechanische Eigenschaften für ein erfindungsgemäßes Implantat aufweisen können, da somit ein großer Gestaltungsfreiraum bei der Herstellung ermöglicht wird. Überraschend hat sich gezeigt, dass ein Fasergehalt von 40-60 % besonders günstig für die Herstellung und ggf. nachträgliche Formanpassung von erfindungsgemäßen Implantaten ist.

In bestimmten Ausgestaltungsformen der Erfindung kann es vorteilhaft sein, wenn der faserverstärkte thermoplastische Kunststoff Compoundzusätze, bevorzugt Titandioxyd (TiO₂), Magnesiumoxyd, Magnesiumsulfat, Hydroxylapatit (HAP), Tricalciumphosphat (TCP), Bioglas, Calziumsulfat, Zinkphosphat, Zinkoxyd und/oder Magnesiumphosphat, enthält.

Der Begriff "Compoundzusätze" bezeichnet im Rahmen der vorliegenden Erfindung zusätzliche Grundstoffe (Zusatzstoffe, Komposite) wie beispielsweise Füllstoffe, Verstärkungsstoffe oder andere Additive, die dem thermoplastischen Kunststoff zusätzlich zu den verstärkenden Fasern beigemischt werden. Durch die Compoundierung werden Zusatzstoffe mit mindestens einem Grundstoff zu einer homogenen Mischung verbunden.

Den Vorgang zum Herstellen eines compoundierten Kunststoffs nennt man Compoundierung. Die Compoundierung ist somit ein Veredelungsprozess von Kunststoffen durch Beimischung von Zusatzstoffen zur gezielten Optimierung der Eigenschaftsprofile von Kunststoffen. Die Compoundierung erfolgt überwiegend in Extrudern (hauptsächlich gleichläufige Doppelschneckenextruder, aber auch gegenläufige Doppelschneckenextruder, sowie durch Planetwalzenextruder und Ko-Kneter) und umfasst z.B. Verfahrensoperationen wie Fördern, Aufschmelzen, Dispergieren, Mischen, Entgasen und Druckaufbau. Im Fall von Thermoplasten kann die Compoundierung z.B. mit Hilfe von Doppelschneckenextrudern erfolgen.

Die Ziele der Compoundierung sind vielfältig und richten sich nach den gewünschten Eigenschaften des späteren Kunststoffteils. Ein wichtiges Ziel der Compoundierung im Rahmen der vorliegenden Erfindung ist insbesondere die Farbeinstellung des faserverstärkten thermoplastischen Kunststoffs, aus dem das erfindungsgemäße Implantat hergestellt wird. Dies kann über die Zugabe von Pigmentpartikeln zum faserverstärkten thermoplastischen Kunststoff erfolgen.

Zudem können die mechanischen Eigenschaften von faserverstärkten thermoplastischen Kunststoffen durch die Zugabe von Compounds, auch durch farbgebende Pigment-Compounds, verändert werden. Compounds können zudem als Verarbeitungshilfsstoffe eingesetzt werden, um z.B. die Entformung der jeweiligen thermoplastischen Kunststoffe im Spritzgießprozess zu vereinfachen.

Additive, auch Hilfsstoffe oder Zusatzstoffe genannt, sind Stoffe, die Kunststoffen zugesetzt werden, um bestimmte Eigenschaften zu erreichen oder zu verbessern oder zu modifizieren. Additive werden eingesetzt, um einen positiven Effekt auf Herstellung, Lagerung, Verarbeitung oder Produkteigenschaften während und nach der Gebrauchsphase zu erreichen. Additive sind in der Regel auf die jeweilige Anwendung hin optimiert.

In bestimmten Ausführungsformen der vorliegenden Erfindung können Coumpoundzusätze beispielsweise dazu verwendet werden, die Knochenintegration des Implantats zu verbessern, eine verminderte Plaqueaffinität zu erzielen oder eine bakterienabweisende Zusammensetzung bereitzustellen.

Solche Compoundzusätze können im Rahmen der Erfindung insbesondere ausgewählt sein aus einer Gruppe umfassend Hydroxylapatit, Magnesiumoxyd, Magnesiumsulfat,SiO₂, BaSO₄, Tricalciumphosphat, Tetracalciumphosphat, Biogläser (resorbierbare und/oder stabile Calciumphosphatkeramiken wie z.B. GB9, GB14, AP40) und TiO₂. Der thermoplastische Kunststoff kann insbesondere mit pulverförmigen Compoundzusätzen versetzt sein, wie beispielsweise Hdroxylapatit, Tricalciumphosphat, Tetracalciumphosphat, Bariumsulfat, Titandioxid, Zinkoxid und Biogläser (resorbierbare und/oder stabile Calciumphosphatkeramiken wie z.B. GB9, GB14, AP40).

In einer besonders bevorzugten Ausführungsform der vorliegenden Erfindung umfasst der faserverstärkte thermoplastische Kunststoff den Compoundzusatz Titandioxid. Die Beimischung von Titandioxid ist in mehrfacher Hinsicht vorteilhaft. Zum einen erhält das erfindungsgemäße Implantat, selbst wenn es mit dunklen Kohlefasern verstärkt ist, eine helle Farbe, die der von Zähnen ähnelt. Zum anderen erhöht Titandioxid die Biokompatibilität von thermoplastischen Kunststoffen, insbesondere von PEEK, und fördert die Einheilung des Implantats in den Knochen.

Die Beimischung von HAP führt ebenfalls zu einer verbesserten Knocheneinheilung eines entsprechenden Implantats. Compoundzusätze wie BaSO₄ können zudem zu einer Erhöhung Röntgenopazität führen.

In einer Ausführungsform der vorliegenden Erfindung machen die Compoundzusätze 0-20 % des Gesamtgewichts (Gewichtsprozent, % (w/w)) des faserverstärkten thermoplastischen Kunststoffs aus, vorzugsweise 5-15 % (w/w), besonders bevorzugt 7-12 % (w/w), insbesondere 10 % (w/w). Dabei haben die pulverförmigen Compoundzusätze eine Partikelgröße von etwa 0,1-100 µm, bevorzugt von etwa 0,1-10 µm, besonders bevorzugt von etwa 0,5-5 µm. Partikel im Nanometer-Bereich haben den Vorteil, dass sie aufgrund ihrer maximalen Oberflächenvergrößerung eine erhöhte biologische Wirksamkeit haben, wobei sie aufgrund ihrer geringen Partikelgröße die mechanischen Eigenschaften der Kunststoffmatrix nur minimal beeinflussen.

In bevorzugten Ausführungsform des erfindungsgemäßen Implantats enthält der faserverstärkte thermoplastische Kunststoff nur im Oberflächenbereich des Implantats bis zu einer Tiefe von bis zu 200 µm Compoundzusätze.

Unter "Oberflächenbereich" im Sinne der vorliegenden Erfindung ist die äußere Schicht des erfindungsgemäßen Implantats zu verstehen, welche die äußere Oberfläche des Implantats umfasst, sowie den sich von der äußeren Oberflache des Implantats nach innen ausdehnende Bereich des Implantats. Der so definierte Oberflächenbereich bildet somit die äußere Ummantelungsschicht des Implantats und hat eine Schichtdicke von bis zu 200 µm. Diese Schichtdicke wird im Rahmen der vorliegenden Erfindung auch als "Tiefe" bezeichnet.

Es ist besonders vorteilhaft, das die Oberfläche eines erfindungsgemäßen Implantats durch die Beimischung von Compoundzusätzen, wie beispielsweise Titandioxid, so verändert werden kann, dass beispielsweise die Einheilung des Implantats in den Knochen erleichtert wird, die Farbe des Implantats nach den Wünschen des Patienten angepasst werden kann oder die Röntgenopazität des Implantats vorteilhaft verändert wird, ohne dass hierbei die mechanischen Eigenschaften des Implantats verändert werden.

Es hat sich gezeigt, das Implantate, die aus zwei miteinander verbundenen Kunststoffen-Komponenten gefertigt sind, wobei eine erste Kunststoff-Komponente mit einem E-Modul ähnlich dem von Knochen außen liegt und eine Schichtdicke von > 200 µm hat, und eine zweite innenliegende Kunststoff-Komponente einen höheren E-Modul aufweist und dem Implantat die nötige Stabilität verleiht, aufgrund der erhöhten Elastizität der äußeren Schicht eine nachteilige Lastenverteilung auf den umliegenden Knochen induzieren. Hierbei entstehen aufgrund der starken elastischen Eigenverformung der äußeren Schicht übermäßige Bewegungen am Knochen-Implantat-Interface, was eine Osseointegration behindert und zu einem Implantatverlust führen kann. Ein Beispiel hierfür ist in der speziellen Beschreibung der Erfindung erläutert.

Überraschenderweise hat sich aber gezeigt, dass eine Veränderung der Zusammensetzung des Materials eines erfindungsgemäßen Implantats im Oberflächenbereich bis zu einer Tiefe von etwa 200 µm zu keiner Veränderung der mechanischen Eigenschaften des Implantats führt im Vergleich zu einem identischen Implantat ohne diese Veränderung im Oberflächenbereich.

Hieraus ergibt sich der überraschende Vorteil, dass der Hersteller eines erfindungsgemäßen Implantats bei der Auswahl der Compoundzusätze, die ausschließlich im Oberflächenbereich des erfindungsgemäßen Implantats zugesetzt werden sollen, um beispielsweise die Einheilung des Implantats in den Knochen zu fördern oder die äußere Farbe des Implantats anzupassen, eine besonders große Auswahl und Flexibilität hat, da er nicht befürchten bzw. berücksichtigen muss, dass der jeweilige Compoundzusatz sich nachteilig auf die mechanischen Eigenschaften des Implantats auswirken könnte. Dies lässt dem Anwender eine größere Freiheit in der Zusammensetzung des Materials des Oberflächenbereichs, um hier die optimalen Eigenschaften für die jeweilige Anwendung zu erzielen.

In einer bevorzugten Ausführungsform des erfindungsgemäßen Implantats handelt es sich um ein Zahnimplantat.

Gemäß einer weiteren bevorzugten Ausführungsform der vorliegenden Erfindung ist es zweckmäßig, wenn das erfindungsgemäße Implantat durch ein 3D-Druckverfahren und/oder Lasersinterverfahren hergestellt worden ist.

3D-Druck und insbesondere Lasersinterverfahren ermöglichen die Fertigung von erfindungsgemäßen Implantaten aus faserverstärktem Kunststoff, wobei auch die Verarbeitung von Lang- und Endlosfasern möglich ist.

Beim 3D-Druck werden erfindungsgemäße Implantate schichtweise aufgebaut. Der Aufbau erfolgt computergesteuert aus einem oder mehreren flüssigen oder festen Werkstoffen nach vorgegebenen Maßen und Formen (Computer Aided Design / CAD). Beim Aufbau finden physikalische oder chemische Härtungs- oder Schmelzprozesse statt. Typische Werkstoffe für das 3D-Drucken sind Kunststoffe, Kunstharze, Keramiken und Metalle. Zudem können durch 3D-Druck thermoplastische Kunststoffe und insbesondere Hochleistungskunststoffe wie PEEK verwendete werden, wobei diese gleichzeitig variabel mit Fasern und Compoundzusätzen versehen werden können.

Erfindungsgemäße Implantate, die mittels 3D-Druck hergestellt wurden, haben gegenüber Implantaten, die durch Spritzguss hergestellt wurden, den Vorteil, dass das aufwendige Herstellen von Formen und das Formenwechseln entfallen. Zudem besteht gegenüber Implantaten, die durch abtragende Verfahren, wie beispielsweise Schneiden, Drehen, Fräsen, Spanabtrag oder Bohren, hergestellt worden sind, der Vorteil, dass es beim 3D-Drucken nicht zu einem Materialverlust kommt, was wesentlich effizienter und gleichzeitig kostensparend ist. Zudem bleiben Lang- und Endlosfasern, die in den thermoplastischen Kunststoff des erfindungsgemäßen Implantats eingearbeitet werden, beim 3D-Druck unbeschädigt, wohingegen diese bei abtragenden Herstellungsverfahren durchtrennt werden, was sich negativ auf die mechanischen Eigenschaften des erfindungsgemäßen Implantats auswirkt.

Zudem ist der Vorgang des 3D-Druckens energetisch vorteilhaft gegenüber anderen Herstellungsverfahren, weil das Material nur einmal in der benötigten Größe und Masse aufgebaut wird. Eine weitere Stärke des 3D-Drucks ist die Möglichkeit, die mitunter komplexen Formen erfindungsgemäßer Implantate aufzubauen, die mit existierenden Maschinen schwer oder gar nicht herstellbar sind. Durch 3D-Druck können auch individualisierte Implantate, die nur für eine bestimmte, einmalige Anwendung benötigt werden, kosteneffizient und schnell hergestellt werden, was ein großer Vorteil gegenüber anderen Herstellungsverfahren ist.

Durch 3D-Druck hergestellte erfindungsgemäße faserverstärkte Implantate haben den Vorteil, dass die Grundgerüste aufgrund der Möglichkeit der präzisen Orientierung der Faserverstärkung sehr grazil gestaltet werden können. Zudem kann ein erfindungsgemäßes Implantat bei der Herstellung durch ein 3D-Druckverfahren direkt mit Compoundzusätzen (auch "Verblendkomposit" genannt) im Oberflächenbereich bedruckt werden, was zu einer spaltfreien Verbindung zwischen dem Verblendkomposit und dem Kunststoff führt. Die Haftung zwischen den beiden Materialien kann zusätzlich durch eine in den Drucker integrierte Plasma-Behandlung der Kunststoff-Oberfläche verstärkt werden. Eine entsprechend integrierte Plasma-Behandlung kann ebenso dazu dienen, die Oberflächen der eingebrachten Fasern zu aktivieren, um den Faser-Matrix-Verbund zu verbessern.

Techniken des 3D-Druckens umfassen, ohne hierauf limitiert zu sein, das selektive Laserschmelzen, das Elektronenstrahlschmelzen, das selektive Lasersintern, die Stereolithografie, das Digital Light Processing für flüssige Kunstharze und das Polyjet-Modeling sowie das Fused Deposition Modeling.

Lasersintern im Sinne der vorliegenden Erfindung ist ein spezifisches 3D-Druckverfahren, bei dem räumliche Strukturen durch Sintern aus einem pulverförmigen Ausgangsstoff hergestellt werden.

Lasersinterverfahren können zur Herstellung von erfindungsgemäßen Implantaten genutzt werden. Dabei werden feinkörnige Werkstoffe, insbesondere thermoplastische Kunststoffe, verstärkende Fasern und Compoundzusätze erhitzt, wobei auch ein erhöhter Druck eingesetzt werden kann. Lasersintern ist ein generatives Schichtbauverfahren, bei dem das erfindungsgemäße Implantat Schicht für Schicht aufgebaut wird. Durch die Wirkung der Laserstrahlen können so beliebige dreidimensionale Geometrien auch mit Hinterschneidungen erzeugt werden, so dass auch komplex geformte Implantate hergestellt werden können, die sich in konventioneller mechanischer oder gießtechnischer Fertigung nicht herstellen lassen.

Grundvoraussetzung ist, dass die dreidimensionalen Geometriedaten des herzustellenden Implantats vorliegen und als Schichtdaten verarbeitet sind. Beispielsweise werden aus vorliegenden CAD-Daten des herzustellenden Implantats (üblicherweise im STL-Format) durch sogenanntes "Slicen" zahlreiche Schichten erzeugt.

Die pulverförmigen Werkstoffe werden auf eine Bauplattform mit Hilfe einer Rakel oder Walze vollflächig in einer Dicke von 1-200 µm aufgebracht. Die Schichten werden durch eine Ansteuerung des Laserstrahles entsprechend der Schichtkontur des Bauteils schrittweise in das Pulverbett gesintert oder eingeschmolzen. Die Bauplattform wird nun geringfügig abgesenkt und eine neue Schicht aufgezogen. Das Pulver wird durch Anheben einer Pulverplattform oder als Vorrat in der Rakel zur Verfügung gestellt. Die Bearbeitung erfolgt Schicht für Schicht in vertikale Richtung, wodurch es möglich ist, auch hinterschnittene Konturen am erfindungsgemäßen Implantat zu erzeugen. Die Energie, die vom Laser zugeführt wird, wird vom Pulver absorbiert und führt zu einem lokal begrenzten Sintern von Partikeln unter Reduktion der Gesamtoberfläche.

Bei den verwendeten Kunststoffpulvern ist es üblich, diese nicht durch Mahlen herzustellen, sondern direkt als Kügelchen zu polymerisieren, da im Prozess sehr hohe Anforderungen an die Beschaffenheit, wie z. B. die Rieselfähigkeit, des verwendeten Pulvers gestellt werden.

Bei der Herstellung von erfindungsgemäßen Implantaten durch ein 3D-Druckverfahren und/oder Lasersinterverfahren ist es möglich, die verstärkenden Fasern gezielt auszurichten und lokal zu bündeln und/oder zu verflechten, um diese optimal an die mechanischen Anforderungen anzupassen. Es ist möglich sowohl kontinuierliche Fasern, Endlosfasern, Langfasern und/oder Kurzfasern in den thermoplastischen Kunststoff gezielt mit einer bestimmten Orientierung und bereichsweise unterschiedlich einzuarbeiten. Die Auswahl, Anordnung und Beschaffenheit der verdruckten Fasern kann sich nach einer zuvor ermittelten Zusammensetzung und Orientierung richten, die eine optimale Verstärkung des Implantats gewährleistet. Die dabei zugrunde liegende Druckstrategie kann zusätzlich durch eine in X-, Y- und Z-Richtung kippbare und um ihre Achse drehbare Basis unterstützt werden, wobei der Druckkopf in alle Achsrichtungen beweglich ist, um den idealen Fasereintrag zusammen mit der Kunststoffmatrix zu gewährleisten. Hierdurch kann eine gezielt multidirektionale Faserausrichtung erzielt werden.

So können speziell beanspruchte Bereiche des Implantats, wie z.B. ein Innengewinde, das für die Verankerung von Implantataufbauten genutzt wird, oder ein Außengewinde, das für die Verankerung des Implantats im Knochen genutzt wird, durch spezifische Anordnung von Fasern oder die bereichsspezifische Anpassung der Faserdichte verstärkt werden. Das 3D-Druckverfahren ermöglicht zudem die simultane Beimischung von Compoundzusätzen, insbesondere auch nur in bestimmten Bereichen, wie beispielsweisem dem Oberflächenbereich des Implantats.

Im Gegensatz zu anderen Herstellungsverfahren ermöglich 3D-Druck die individualisierte und zeiteffiziente Herstellung von Implantaten, auch auf Basis von individuellen Datensätzen, die Informationen über die räumliche Umgebung des Implantats und die dort wirkenden Kräfte bereitstellen. Hierbei kann die Faserorientierung gezielt bestimmt werden, wodurch insbesondere eine multidirektionale, orientierte und/oder auf die bereichsweise Belastung des Implantats abgestimmte Orientierung der Fasern ermöglicht wird.

Entsprechende 3D-Drucker sind dem Fachmann bekannt. Sie enthalten Mischersysteme, die es ermöglichen, mehrere Polymerkomponenten in verschiedenen Verhältnissen dem Druckkopf zuzuführen (Gradientenverfahren). So können in einem Druckvorgang verschiedene mechanische und physikalische Eigenschaften innerhalb eines Bauteiles verwirklicht werden.

Ein großer Vorteil der Herstellung von Implantaten durch 3D-Druck ist, dass es möglich ist individualisierte Implantate für den Patienten direkt am Behandlungsort, beispielsweise einem Krankenhaus oder einer Arztpraxis, mit Hilfe eines dort vorhandenen 3D-Druckers herzustellen. Nachdem die individuellen Patientendaten, insbesondere in Bezug auf die räumliche Umgebung des Implantats, die Knochendichte in den an das Implantat angrenzenden Bereichen, und die auf das Implantat einwirkenden Kräfte, wie z.B. Kaukräfte im Fall von Zahnimplantaten, erhoben wurden, können diese dazu genutzt werden ein individualisiertes Implantat durch 3D-Druck vor Ort herzustellen und direkt einzusetzen. So ist es beispielsweise möglich ein erfindungsgemäßes individualisiertes Zahnimplantat für einen Patienten innerhalb einer Sitzung beim Zahnarzt herzustellen und einzusetzen.

In einer weiteren bevorzugten Ausführungsform der Erfindung wurde das erfindungsgemäße Implantat durch ein CFM-Verfahren oder ein Formpressverfahren hergestellt.

Das CFM-Verfahren (CFM = composite flow molding) ist ein Hochdruck-Formverfahren, bei dem ein, meist durch parallel ausgerichtete Lang- oder Endlosfasern verstärkter Verbundwerkstoff (in der Regel ein Teil einer pultrudierten Stange), vorzugsweise ein faserverstärkter thermoplastischer Kunststoff, besonders bevorzugt kohlefaserverstärktes PEEK, durch Hitze und Druck direkt umgewandelt wird in die Form des herzustellenden Gegenstandes, beispielsweise ein erfindungsgemäßes Implantat.

Beim CFM-Verfahren wird beispielsweise ein faserverstärktes PEEK-Halbzeug, das durch ein Pultrusionsverfahren hergestellt wurde, durch Hitzeeinwirkung zum Schmelzen gebracht und durch einen Stempel in eine Gussform gepresst. Dabei kommt es zu einer zufällig multidirektionalen Anordnung der Fasern im hergestellten Bauteil. Bei kleinen Bauteilen, wie beispielsweise Zahnimplantaten, kommen die positiven mechanischen Effekte, die sich aus der multidirektionalen Anordnung der Fasern in Bezug auf die Stabilität des Bauteils ergeben, besonders zum Tragen.

Anders als beispielsweise beim Spritzgießen, ist es beim CFM-Verfahren möglich, faserverstärkte thermoplastische Kunststoffe, wie beispielsweise PEEK, mit Endlosfasern zu verarbeiten, wodurch das hergestellte Implantat vorteilhafte Eigenschaften bzgl. seiner Festigkeit und seines Elastizitätsmoduls aufweist. Die Fasern ordnen sich zufällig innerhalb der Schraube an, so dass eine multidirektionale Faserausrichtung gewährleistet wird.

Überraschenderweise kommt es aber zusätzlich zu einer spezifischen, kontinuierlichen Ausrichtung der Fasern entlang der Außenkonturen des Implantats, beispielsweise auch innerhalb von Gewindestrukturen. Dadurch erhält ein solches Implantat eine besonders hohe Zugfestigkeit und Stabilität beispielsweise im Bereich von ggf. vorhandenen Gewindeflanken, was die dauerhafte Stabilität eines solchen Implantats im montierten Zustand gewährleistet. Gleichzeitig sind die Fasern durch ihre feste Einbettung in der Matrix vor Abrieb geschützt.

Das Formpressverfahren ist ein Herstellungsverfahren für Kunststoffe, mit dem faserverstärkte thermoplastische Kunststoffe verarbeitet werden können. Zu Beginn des Verfahrens wird der faserverstärkte thermoplastische Kunststoff in eine Kavität eingebracht, welche aufgeheizt wird um den Kunststoff zu schmelzen. Anschließend wird die Kavität unter Einsatz eines Druckkolbens geschlossen. Durch den Druck erlangt der Kunststoff die von der Kavität des Formwerkzeugs vorgegebene Form, beispielsweise die eines Implantats. Nach dem Abkühlen kann das Bauteil aus dem Formwerkzeug entnommen und ggf. nach- oder weiterverarbeitet werden.

Es war überraschend, dass so feine Bauteile wie Implantate, insbesondere Zahnimplantate, aus faserverstärktem thermoplastischem Kunststoff durch Formpressen hergestellt werden können. Implantate, die mittels Formpressen hergestellt werden, haben den Vorteil, dass sie schnell und in hoher Stückzahl produziert werden können. Zudem weisen durch Formpressen hergestellte Implantate eine multidirektionale Faseranordnung auf, was zu einer besonderen mechanischen Widerstandsfähigkeit und Stabilität der Implantate beiträgt. Überraschenderweise führt die Herstellung durch Formpressen zu einer besonders hohen Stabilität von am Implantat vorhandenen Gewindeflanken, die ansonsten besonders instabil unter Belastung sind.

In einer weiteren bevorzugten Ausführungsform der Erfindung wurde das erfindungsgemäße Implantat durch ein Flechtverfahren hergestellt, wobei die verstärkenden Fasern als geflochtene Faseranordnung auf einem formgebenden Kern abgelegt werden und anschließend von Kunststoff umschlossen werden. Hierbei können mehrere Schichten von geflochtenen Langfasern jeweils nacheinander auf einem formgebenden Kern aus PEEK abgelegt werden, so dass das Implantat schichtweise wächst und jede Schicht eine spezifische Faseranordnung aufweist. Von einem kleinen formgebenden Kern ausgehend kann das Implantat schichtweise aufgebaut werden, indem abgelegte geflochtene Faserschichten von einer PEEK-Schicht umschlossen werden, auf die dann die nächste Schicht geflochtener Fasern abgelegt werden kann.

Durch dieses Verfahren hergestellte Implantate sind besonders vorteilhaft, da jede Flechtschicht eine spezifische Faseranordnung aufweisen kann und eine bereichsweise Anpassung der mechanischen Eigenschaften ermöglicht wird.

Mit der Flechttechnik können komplexe Strukturen, wie feinste chirurgische Nähfäden oder medizinische Stents, hergestellt werden. Der Fadenwinkel beim Flechten kann voreingestellt werden. Das ermöglicht maßgeschneiderte Flechtprodukte. Die Flechttechnik ist eine wirtschaftliche Fertigungsmethode, die gut automatisierbar ist und eine hohe Flexibilität bietet. Da das Geflecht konturnah auf einem formgebenden Kern abgelegt werden kann, ist das Flechtverfahren eine sehr verschnittarme und quasi drapierfreie Technologie. Darüber hinaus profitiert sie von den einstellbaren Fadenwinkeln zur Aufnahme von Torsions- und Schublasten.

In einer weiteren bevorzugten Ausführungsform der Erfindung wurde das erfindungsgemäße Implantat mit Hilfe eines Filament-Winding-Verfahren hergestellt. Durch das Filament-Winding-Verfahren können erfindungsgemäße Implantate, die mit kontinuierlichen Lang- oder Endlosfasern verstärkt sind, hergestellt werden. Bei diesem Verfahren wird erhitztes Pre-Preg aus vorimprägnierten Fasern beispielsweise in einem überlappenden helikalen Muster mehrere Male um einen rotierenden Metalldorn gewickelt, um einen geschichteten Zylinder mit einem bevorzugten Faseraufbau zu erzeugen. Es ist auch möglich, die Faseranordnung während des Umwickelns gezielt an die jeweilige Position innerhalb des Implantats anzupassen.

Die Pre-Preg wird dabei auf eine Temperatur oberhalb der Schmelztemperatur des verwendeten thermoplastischen Kunststoffs erhitzt und mit Hilfe eines Applikationskopfes mit dem zugrundeliegenden umwickelten Material verbunden. Wenn die angestrebte Anzahl von Faserschichten in der angestrebten, zuvor bestimmten Orientierung und Positionierung aufgetragen wurde, wird der Dorn durch Herausziehen entfernt, so dass ein erfindungsgemäßes Hohlzylinderimplantat aus gezielt faserverstärktem thermoplastischem Kunststoff vorliegt. Es ist auch möglich, ein zuvor gefertigtes zylinderförmiges Bauteil, das den Kern eines erfindungsgemäßen Implantats bilden soll, als Dorn im Filament-Winding-Verfahren zu verwenden, der nicht nachträglich entfernt wird. Umwicklung mit Pre-preg Fasern schafft eine Verstärkung, die Widerstandsfähigkeit gegen Torsionskräfte gewährleistet und Steifigkeit verleiht. Zudem wird die Spaltung des Implantats verhindert, wenn es ungünstig belastet wird, z.B. wenn es von außen angebohrt wird.

Die Erfindung betrifft ein System zur Herstellung eines erfindungsgemäßen Implantats, umfassend
- eine Vorrichtung zur Erfassung von Patientendaten bzgl. der Umgebung, in die ein Implantat eingesetzt werden soll, umfassend
   ∘ die Krafteinwirkung bei mechanischer Belastung,
   ∘ die Knochendichte und/oder
   ∘ die räumliche Struktur der Umgebung,
- ein Computerprogramm zur Erstellung eines Modells für ein individualisiertes Implantat auf Basis der erfassten Patientendaten, wobei
   ∘ die dreidimensionale Struktur des Implantats,
   ∘ die Materialzusammensetzung umfassend einen thermoplastischen Kunststoff, verstärkende Fasern und/oder Compoundzusätze, und
   o die Anordnung der verstärkenden Fasern und der Compoundzusätze berechnet werden,
- eine Vorrichtung zur Herstellung des individualisierten Implantats auf Basis des berechneten Modells mittels 3D-Druck und/oder Lasersintern.

In einer bevorzugten Ausführungsform betrifft die Erfindung ein System zur Herstellung eines erfindungsgemäßen Implantats mit einem Elastizitätsmodul E von 30-50 GPa, umfassend
- eine Vorrichtung zur Erfassung von Patientendaten bzgl. der Umgebung, in die ein Implantat eingesetzt werden soll, wobei die erfassten Patientendaten
   ∘ die Krafteinwirkung bei mechanischer Belastung,
   ∘ die Knochendichte und/oder
   ∘ die räumliche Struktur der Umgebung umfassen,
- ein Computerprogramm zur Erstellung eines Modells für ein individualisiertes Implantat auf Basis der erfassten Patientendaten, wobei
   ∘ eine dreidimensionale Struktur des Implantats,
   ∘ eine Materialzusammensetzung umfassend einen thermoplastischen Kunststoff, verstärkende Langfasern mit einem Durchmesser von 4 bis 10 µm und optional Compoundzusätze, und
   ∘ eine multidirektionale Anordnung der verstärkenden Langfasern und optional der Compoundzusätze berechnet werden, und
- eine Vorrichtung zur Herstellung des individualisierten Implantats auf Basis des berechneten Modells mittels 3D-Druck und/oder Lasersintern.

Unter einem "System" im Sinne der vorliegenden Erfindung ist eine Gesamtheit von Elementen zu verstehen, die zur Herstellung eines individuellen Implantats benötigt werden, die so aufeinander bezogen oder miteinander verbunden sind und in einer Weise interagieren, dass sie als Einheit zur Herstellung eines erfindungsgemäßen Implantats angesehen werden können.

Die Vorrichtung zur Erfassung von Patientendaten kann beispielsweise ein Messinstrument sein, mit dessen Hilfe die Patientendaten erhoben und gespeichert werden. In einer anderen Ausführungsform kann diese Vorrichtung auch ein Datenträger sein, auf dem Daten, die zuvor mit anderen Vorrichtungen erhoben wurden, gespeichert und somit erfasst werden. Solche Vorrichtungen umfassen insbesondere Computerfestplatten, aber auch andere computerlesbare Speichermedien oder Datenträger.

Die von der Vorrichtung zur Erfassung von Patientendaten bereitgestellten Daten werden innerhalb des erfindungsgemäßen Systems von einem Computerprogramm verarbeitet, wodurch auf Basis dieser Daten ein Modell eines patientenspezifischen erfindungsgemäßen Implantats erstellt wird. Ein solches Modell beschreibt die dreidimensionale Struktur des Implantats, wobei zur Erstellung dieser Struktur die Patientendaten bzgl. der räumlichen Struktur der Umgebung, in die das Implantat eingesetzt werden soll, berücksichtigt werden. Zudem wird die Materialzusammensetzung umfassend einen thermoplastischen Kunststoff, was auch einen Blend aus zwei oder mehr Kunststoffen mit einschließt, verstärkende Fasern und Compoundzusätze in dem erstellten Modell berücksichtigt. Das Computerprogramm berücksichtigt bei der Auswahl der Materialzusammensetzung die erfassten Patientendaten, um die für die individuelle Situation, in der das herzustellende Implantat verwendet werden soll, optimale Zusammensetzung zu berechnen.

In dem vom Computerprogramm erstellten Modell des Implantats wird zudem die Positionierung und Orientierung der verstärkenden Fasern innerhalb des Implantats berücksichtigt. Dies schließt insbesondere die Verteilung der verstärkenden Fasern, die Faserzusammensetzung, die Faserdurchmesser und/oder die Faserdichte in den verschiedenen Bereichen des Implantats ein. Auf Basis der Patientendaten kann von dem Computerprogramm berechnet werden, in welchem Bereich des Implantats eine besondere Verstärkung für eine Erhöhung der Stabilität notwendig ist, und in welchen Bereichen es vorteilhaft ist, weniger verstärkende Materialien einzubauen, um die Flexibilität zu erhöhen.

Weiterhin berücksichtigt das Computerprogramm bei der Erstellung des Implantatmodells, welche Compoundzusätze verwendet werden sollen und in welchen Bereichen des Implantats diese eingesetzt werden sollen. Compoundzusätze können im gesamten Implantat verarbeitet werden, um die Gesamteigenschaften des Implantats zu beeinflusse. Alternativ können bestimmte Compoundzusätze nur im Oberflächenbereich des Implantats eingesetzt werden, um beispielsweise eine Farbabdeckung zu erzeugen oder die Knocheneinheilung des Implantats nach dem Einsetzten zu erleichtern, ohne hierbei die mechanischen Eigenschaften des verwendeten faserverstärkten thermoplastischen Kunststoffs zu verändern.

Als Vorrichtung zur Herstellung des individualisierten Implantats auf Basis des berechneten Modells kann jede Art von 3D-Drucker verwendet werden, insbesondere solche, die Fused Deposition Modeling (FDM), Stereolithographie (SLA), Multi Jet Modeling (MJM), Selektives Lasersintern (SLS), Selektives Laserschmelzen (SLM), Selektives Elektronenstrahlschmelzen (SEBM) und/oder 3-Dimensional Printing (3DP von Zcorp.) anwenden.

Das erfindungsgemäße System hat den Vorteil, dass innerhalb kurzer Zeit ein individualisiertes erfindungsgemäßes Implantat hergestellt werden kann, das an die patientenspezifischen Anforderungen angepasst ist. Hierdurch können die Nachteile bestehender Implantatlösung weitestgehend vermieden werden. Insbesondere negative mechanische Belastungen für den angrenzenden Knochen und die Bruchgefahr können so minimiert werden. Zudem kann durch den gezielten Einsatz von Compoundzusätzen, insbesondere im Oberflächenbereich des Implantats, eine gezielte farbliche Anpassung des Implantats erfolgen, die Plaqueadhärenz vermindert werden und eine bakterienabweisende Oberfläche eingearbeitet werden. Weiterhin kann die mechanische Beschaffenheit des Implantats so gestaltet werden, dass eine bakteriendichte Verbindung zu mit dem Implantat zu verbindenden Bauteilen möglich ist.

Mitunter können mit Hilfe dieses Systems sämtlich PEEK-basierten Zahnersatzvarianten von einer Einzelzahnkrone über eine Brückenkonstruktion bis hin zu gesamten Teil- und Vollprothesen hergestellt werden. Insbesondere mit einem zusätzlichen Druckkopf speziell für Verblendkomposite und/oder Compoundzusätze können faserverstärkten PEEK-Gerüste mit einem aufdruckbaren dentalen Verblendkomposit bzw. Compoundzusätzen verblendet werden, so dass ihnen das Erscheinungsbild natürlicher Zähne verliehen werden kann. Zudem betrifft die vorliegende Erfindung eine Vorrichtung zur Herstellung eines erfindungsgemäßen Implantats, umfassend
- eine Einrichtung zur Erfassung von Patientendaten bzgl. der Umgebung, in die ein Implantat eingesetzt werden soll, umfassend
   ∘ die Krafteinwirkung bei mechanischer Belastung,
   ∘ die Knochendichte und/oder
   ∘ die räumliche Struktur der Umgebung
- eine Einrichtung zur Erstellung eines Modells für ein individualisiertes Implantat auf Basis der erfassten Patientendaten, wobei
   ∘ die dreidimensionale Struktur des Implantats,
   ∘ die Materialzusammensetzung umfassend einen thermoplastischen Kunststoff, verstärkende Fasern und/oder Compoundzusätze, und
   ∘ die Anordnung der verstärkenden Fasern und der Compoundzusätze berechnet werden,
- eine Einrichtung zur Herstellung des individualisierten Implantats auf Basis des berechneten Modells mittels 3D-Druck und/oder Lasersintern.

Bevorzugt betrifft die Erfindung eine Vorrichtung zur Herstellung eines erfindungsgemäßen Implantats mit einem Elastizitätsmodul E von 30-50 GPa, umfassend
- eine Einrichtung zur Erfassung von Patientendaten bzgl. der Umgebung, in die ein Implantat eingesetzt werden soll, wobei die erfassten Patientendaten
   ∘ die Krafteinwirkung bei mechanischer Belastung,
   ∘ die Knochendichte und/oder
   ∘ die räumliche Struktur der Umgebung umfassen,
- eine Einrichtung zur Erstellung eines Modells für ein individualisiertes Implantat auf Basis der erfassten Patientendaten, wobei
   ∘ eine dreidimensionale Struktur des Implantats,
   ∘ eine Materialzusammensetzung umfassend einen thermoplastischen Kunststoff, verstärkende Langfasern mit einem Durchmesser von 4 bis 10 µm und optional Compoundzusätze, und
   ∘ eine multidirektionale Anordnung der verstärkenden Langfasern und optional der Compoundzusätze berechnet werden, und
- eine Einrichtung zur Herstellung des individualisierten Implantats auf Basis des berechneten Modells mittels 3D-Druck und/oder Lasersintern.

Erfindungsgemäß ist unter einer Vorrichtung insbesondere ein Gerät, ein Apparat oder eine Einrichtung zu verstehen, die aus verschiedenen Teilgeräten, Vorrichtungen oder Einrichtungen zusammengesetzt sein kann, die zusammen die Herstellung eines erfindungsgemäßen Implantats ermöglichen.

Die erfindungsgemäßen Vorrichtung umfasst ein Teilgerät bzw. eine Einrichtung zur Erfassung von Patientendaten, wie beispielsweise ein Messinstrument, mit dessen Hilfe Patientendaten erhoben und gespeichert werden können, oder auch ein Datenträger, auf dem Daten, die zuvor mit anderen Vorrichtungen erhoben wurden, gespeichert und somit erfasst werden. Solche Einrichtungen umfassen insbesondere Computerfestplatten, aber auch andere computerlesbare Speichermedien oder Datenträger.

Die von der Einrichtung zur Erfassung von Patientendaten bereitgestellten Daten werden innerhalb der erfindungsgemäßen Vorrichtung von einer zweiten Einrichtung zur Erstellung eines Modells für ein individualisiertes Implantat auf Basis der erfassten Patientendaten verwendet, wodurch von dieser Einrichtung auf Basis der Daten ein Modell eines patientenspezifischen erfindungsgemäßen Implantats erstellt wird. Ein solches Modell beschreibt die dreidimensionale Struktur des Implantats, wobei zur Erstellung dieser Struktur die Patientendaten bzgl. der räumlichen Struktur der Umgebung, in die das Implantat eingesetzt werden soll, berücksichtigt werden.

Zudem wird die Materialzusammensetzung, umfassend einen thermoplastischen Kunststoff, was auch einen Blend aus zwei oder mehr Kunststoffen mit einschließt, verstärkende Fasern und Compoundzusätze, in dem erstellten Modell berücksichtigt.

In dem erstellten Modell des Implantats wird zudem die Positionierung und Orientierung der verstärkenden Fasern innerhalb des Implantats berücksichtigt. Dies schließt insbesondere die Verteilung der verstärkenden Fasern, die Faserzusammensetzung, die Faserdurchmesser und/oder die Faserdichte in den verschiedenen Bereichen des Implantats ein. Auf Basis der Patientendaten kann von der Einrichtung zur Erstellung des Modells berechnet werden, in welchem Bereich des Implantats eine besondere Verstärkung für eine Erhöhung der Stabilität notwendig ist, und in welchen Bereichen es vorteilhaft ist, weniger verstärkende Materialien einzubauen, um die Flexibilität bzw. Elastizität zu erhöhen.

Weiterhin wird bei der Erstellung des Implantatmodells durch die erfindungsgemäße Vorrichtung berücksichtig, welche Compoundzusätze verwendet werden sollen und in welchen Bereichen des Implantats diese eingesetzt werden sollen. Compoundzusätze können im gesamten Implantat verarbeitet werden, um die Gesamteigenschaften des Implantats zu beeinflusse. Alternativ können bestimmte Compoundzusätze nur im Oberflächenbereich des Implantats eingesetzt werden, um beispielsweise eine Farbabdeckung zu erzeugen oder die Knocheneinheilung des Implantats nach dem Einsetzten zu erleichtern, ohne hierbei die mechanischen Eigenschaften des verwendeten faserverstärkten thermoplastischen Kunststoffs zu verändern.

Als Vorrichtung zur Herstellung des individualisierten Implantats auf Basis des berechneten Modells kann jede Art von 3D-Drucker verwendet werden, insbesondere solche, die Fused Deposition Modeling (FDM), Stereolithographie (SLA), Multi Jet Modeling (MJM), Selektives Lasersintern (SLS), Selektives Laserschmelzen (SLM), Selektives Elektronenstrahlschmelzen (SEBM) und/oder 3-Dimensional Printing (3DP von Zcorp.) anwenden.

Die erfindungsgemäße Vorrichtung hat den Vorteil, dass innerhalb kurzer Zeit ein individualisiertes erfindungsgemäßes Implantat hergestellt werden kann, das an die patientenspezifischen Anforderungen angepasst ist. Hierdurch können die Nachteile bestehender Implantatlösung weitestgehend vermieden werden. Insbesondere negative mechanische Belastungen für den angrenzenden Knochen und die Bruchgefahr können so minimiert werden. Zudem kann durch den gezielten Einsatz von Compoundzusätzen, insbesondere im Oberflächenbereich des Implantats, eine gezielte farbliche Anpassung des Implantats erfolgen, die Plaqueadhärenz vermindert werden und eine bakterienabweisende Oberfläche eingearbeitet werden. Weiterhin kann die mechanische Beschaffenheit des Implantats so gestaltet werden, dass eine bakteriendichte Verbindung zu mit dem Implantat zu verbindenden Bauteilen möglich ist.

Mitunter können mit Hilfe dieser Vorrichtung sämtlich PEEK-basierten Zahnersatzvarianten von einer Einzelzahnkrone über eine Brückenkonstruktion bis hin zu gesamten Teil- und Vollprothesen hergestellt werden. Insbesondere mit einem zusätzlichen Druckkopf speziell für Verblendkomposite und/oder Compoundzusätze können faserverstärkten PEEK-Gerüste mit einem aufdruckbaren dentalen Verblendkomposit bzw. Compoundzusätzen verblendet werden, so dass ihnen das Erscheinungsbild natürlicher Zähne verliehen werden kann. Weiterhin betrifft die vorliegende Erfindung ein Verfahren zur Herstellung eines erfindungsgemäßen Implantats, umfassend
- die Erfassung von Patientendaten bzgl. der Umgebung, in die ein Implantat eingesetzt werden soll, umfassend
   ∘ die Krafteinwirkung bei mechanischer Belastung,
   ∘ die Knochendichte und/oder
   ∘ die räumliche Struktur der Umgebung
- die Erstellung eines Modells für ein individualisiertes Implantat auf Basis der erfassten Patientendaten, wobei
   ∘ die dreidimensionale Struktur des Implantats,
   ∘ die Materialzusammensetzung umfassend einen thermoplastischen Kunststoff, verstärkende Fasern und/oder Compoundzusätze, und
   ∘ die Anordnung der verstärkenden Fasern und der Compoundzusätze berechnet werden,
- die Herstellung des individualisierten Implantats auf Basis des berechneten Modells mittels 3D-Druck und/oder Lasersintern.

In einer Ausführungsform betrifft die Erfindung ein Verfahren zur Herstellung eines erfindungsgemäßen Implantats mit einem Elastizitätsmodul E von 30-50 GPa, umfassend
- die Erfassung von Patientendaten bzgl. der Umgebung, in die ein Implantat eingesetzt werden soll, wobei die erfassten Patientendaten
   ∘ die Krafteinwirkung bei mechanischer Belastung,
   ∘ die Knochendichte und/oder
   ∘ die räumliche Struktur der Umgebung umfassen,
- die Erstellung eines Modells für ein individualisiertes Implantat auf Basis der erfassten Patientendaten, wobei
   ∘ eine dreidimensionale Struktur des Implantats,
   ∘ eine Materialzusammensetzung umfassend einen thermoplastischen Kunststoff, verstärkende Langfasern mit einem Durchmesser von 4 bis 10 µm und optional Compoundzusätze, und
   ∘ eine multidirektionale Anordnung der verstärkenden Langfasern und optional der Compoundzusätze berechnet werden, und
- die Herstellung des individualisierten Implantats auf Basis des berechneten Modells mittels 3D-Druck und/oder Lasersintern.

Bei dem erfindungsgemäßen Verfahren handelt es sich um ein Verfahren zur Herstellung eines erfindungsgemäßen Implantats. Im Rahmen dieses Verfahrens werden Patientendaten beispielsweise mittels eines Messinstruments erhoben und gespeichert. In einer anderen Ausführungsform des Verfahrens können die Patientendaten auch durch einen Datenträger, auf dem zuvor erhobene Patientendaten gespeichert sind, bereitgestellt und erfasst werden.

Die erfassten Patientendaten werden in einem weiteren Schritt des erfindungsgemäßen Verfahrens zur Erstellung eines Modells für ein individualisiertes Implantat beispielsweise von einem Computerprogramm verwendet. Ein solches Modell beschreibt die dreidimensionale Struktur des Implantats, wobei zur Erstellung dieser Struktur die Patientendaten bzgl. der räumlichen Struktur der Umgebung, in die das Implantat eingesetzt werden soll, berücksichtigt werden.

Zudem wird die Materialzusammensetzung umfassend einen thermoplastischen Kunststoff, was auch einen Blend aus zwei oder mehr Kunststoffen mit einschließt, verstärkende Fasern und Compoundzusätze in dem erstellten Modell berücksichtigt. Weiterhin wird die Positionierung und Orientierung der verstärkenden Fasern innerhalb des Implantats berücksichtigt. Dies schließt insbesondere die Verteilung der verstärkenden Fasern, die Faserzusammensetzung, die Faserdurchmesser und/oder die Faserdichte in den verschiedenen Bereichen des Implantats ein.

Nach Erstellung des Modells des erfindungsgemäßen individualisierten Implantats wird in einem weiteren Schritt des erfindungsgemäßen Verfahrens ein individualisiertes Implantat hergestellt. Hierzu kann jede Art von 3D-Drucker verwendet werden, insbesondere solche, die Fused Deposition Modeling (FDM), Stereolithographie (SLA), Multi Jet Modeling (MJM), Selektives Lasersintern (SLS), Selektives Laserschmelzen (SLM), Selektives Elektronenstrahlschmelzen (SEBM) und/oder 3-Dimensional Printing (3DP von Zcorp.) anwenden.

Das erfindungsgemäße Verfahren hat den Vorteil, dass innerhalb kurzer Zeit ein individualisiertes erfindungsgemäßes Implantat hergestellt werden kann, das an die patientenspezifischen Anforderungen angepasst ist. Hierdurch können die Nachteile bestehender Implantatlösung weitestgehend vermieden werden. Insbesondere negative mechanische Belastungen für den angrenzenden Knochen und die Bruchgefahr können so minimiert werden. Zudem kann durch den gezielten Einsatz von Compoundzusätzen, insbesondere im Oberflächenbereich des Implantats, eine gezielte farbliche Anpassung des Implantats erfolgen, die Plaqueadhärenz vermindert werden und eine bakterienabweisende Oberfläche eingearbeitet werden. Weiterhin kann die mechanische Beschaffenheit des Implantats so gestaltet werden, dass eine bakteriendichte Verbindung zu mit dem Implantat zu verbindenden Bauteilen möglich ist.

Mitunter können mit Hilfe dieses Verfahrens sämtlich PEEK-basierten Zahnersatzvarianten von einer Einzelzahnkrone über eine Brückenkonstruktion bis hin zu gesamten Teil- und Vollprothesen hergestellt werden. Insbesondere mit einem zusätzlichen Druckkopf speziell für Verblendkomposite und/oder Compoundzusätze können faserverstärkten PEEK-Gerüste mit einem aufdruckbaren dentalen Verblendkomposit bzw. Compoundzusätzen verblendet werden, so dass ihnen das Erscheinungsbild natürlicher Zähne verliehen werden kann.

### Spezielle Beschreibung der Erfindung

### Figuren:

Figur 1:
   Längsschnitt durch ein Einkomponenten-Implantat (One Component Implant, 1-C) aus 55-CFR-PEEK und ein Zweikomponenten-Implantat (Two Component Implant, 2-C), das aus einem Implantatkern (Implant Core) aus 55-CFR-PEEK und einer den Kern umgebenden Implantathülle (Implant Shell) aus einem Titandioxid-gefüllten PEEK besteht.
Figur 2:
   Darstellung des Aufbaus der Modelle für ein 1-C und ein 2-C Implantat, die jeweils in ein Knochensegment des Unterkiefers eingebettet sind, das aus einer kortikalen Knochenschicht (Cortical Bone) und einem spongiösen Knochenanteil (Cancellous Bone) besteht, und die mit einem Abutment, einer Abutmentschraube (Abutment screw) und einer Implantatkrone (Implant Crown) ergänzt wurden.
Figur 3:
   Darstellung des überwiegend hexaedrischen Finite-Elemente-Netzes.
Figur 4:
   Darstellung der Einleitung einer 100 N-Last gemäß ISO 14801:2007 in 30°-Richtung zur Längsachse des Implantates (Long axis of the implant).

### Ausführungsbeispiele:

Es wurde eine Finite-Elemente-Analyse durchgeführt, um die mechanischen Auswirkungen auf den periimplantären Knochen bei Belastung eines erfindungsgemäßen, mit zufällig multidirektional ausgerichteten Endloskohlefasern verstärkten PEEK-Implantates (Faseranteil ca. 55 %) im Vergleich zu einem PEEK-Implantat gemäß WO 2014/198421 A1 zu untersuchen.

### Material und Methoden:

Es wurde zwei vereinfachte zylindrische Implantatmodelle erstellt. Das erste Implantatmodell bestand aus nur einer Komponente (one component implant, 1-C), welche aus PEEK, das mit ca. 55 % zufällig multidirektional orientierten Endloskohlefasern (55-CFR-PEEK) verstärkt wurde, gefertigt wurde und einem erfindungsgemäßen Implantat entsprach.

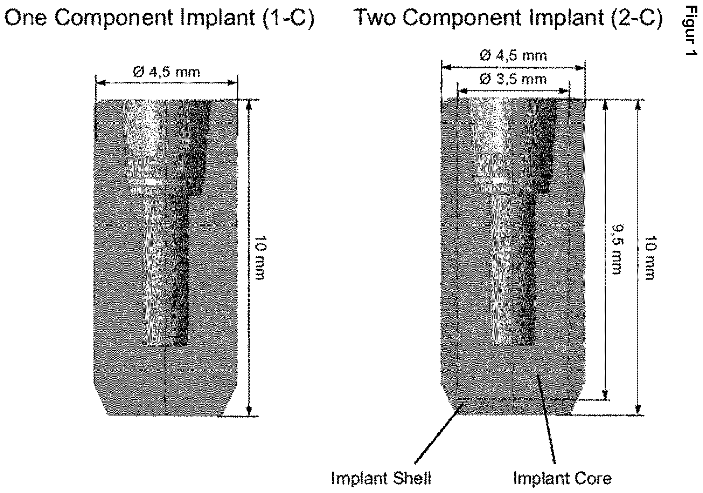

Das zweite Implantatmodell bestand aus zwei Komponenten (two component implant, 2-C): Einem Implantatkern (Implant Core) aus derselben PEEK-Sorte wie Implantatmodell 1-C (55-CFR-PEEK) und einer Implantathülle (Implant Shell), die eine Schichtdicke von 0,5 mm im Oberflächenbereich des Implantats aufwies und aus einer TiO₂-gefüllten PEEK-Sorte bestand. In Figur 1 sind die beiden untersuchten Implantate 1-C und 2-C im Längsschnitt dargestellt.

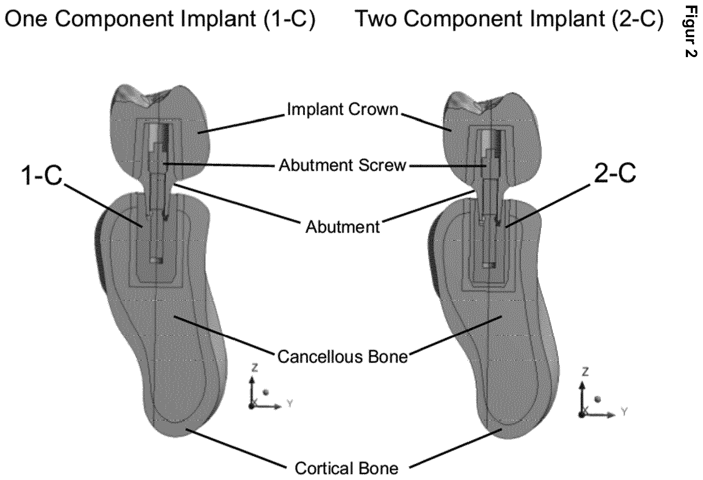

Die beiden Implantate 1-C und 2-C wurden jeweils in ein Knochensegment eingebettet und mit einem Abutment, einer Abutmentschraube und einer Implantatkrone eines unteren ersten Molaren versehen (Figur 2). Die Implantatkrone und das Abutment bestanden aus derselben TiO₂-gefüllten PEEK-Sorte wie die Implantathülle des 2-C-Implantates. Die Abutmentschraube bestand ebenfalls aus 55-CFR-PEEK.

In Tabelle 1 sind die Eigenschaften der verwendeten Materialien zusammengefasst. Für die Analyse wurde angenommen, dass die Materialien isotrop und linear elastisch waren.

**Tabelle 1: Eigenschaften der verwendeten Materialien.**

| **Material** | **E-Modul [GPa]** | **Poisson Ratio** |
|---|---|---|
| TiO₂-gefülltes PEEK (20 % TiO₂) | 4,1 | 0,4 |
| Mit ca. 55 % zufällig multidirektional orientierten Endloskohlefasern verstärktes PEEK (55-CFR-PEEK) | 40 | 0,35 |
| Kortikaler Knochen | 13,4 | 0,3 |
| Spongiöser Knochen | 1,37 | 0,31 |

Anschließend wurden die Verbindungseigenschaften zwischen den Kontaktpaaren der einzelnen Komponenten definiert (Tabelle 2).

**Tabelle 2: Verbindungszustand zwischen den einzelnen Kontaktpaaren.**

| **Verbindung** | **Kontaktpaar** | | **Zustand** |
|---|---|---|---|
| 1 | Krone | Abutment | verschweisst (bonded contact, perfectly bonded) |
| 2 | Schraubenkopf | Abutment | Reibungskontakt (rough contact) |
| 3 | Schraubenkopfunterlag e | Abutment | verschweisst (bonded contact, perfectly bonded) |
| 4 | Schraubenschaft | Abutment | Reibungskontakt (rough contact) |
| 5 | Abutment | Implantat | Reibungskontakt (rough contact) |
| 6 | Schraubengewinde | Implantatinnengewinde | verschweisst (bonded contact, perfectly bonded) |
| 7 | Implantatkern | Implantathülle | verschweisst (bonded contact, perfectly bonded) |
| 8 | Implantathülle | Knochen | Reibungskontakt (rough contact) |
| 9 | Kortikaler Knochen | Spongiöser Knochen | verschweisst (bonded contact, perfectly bonded) |

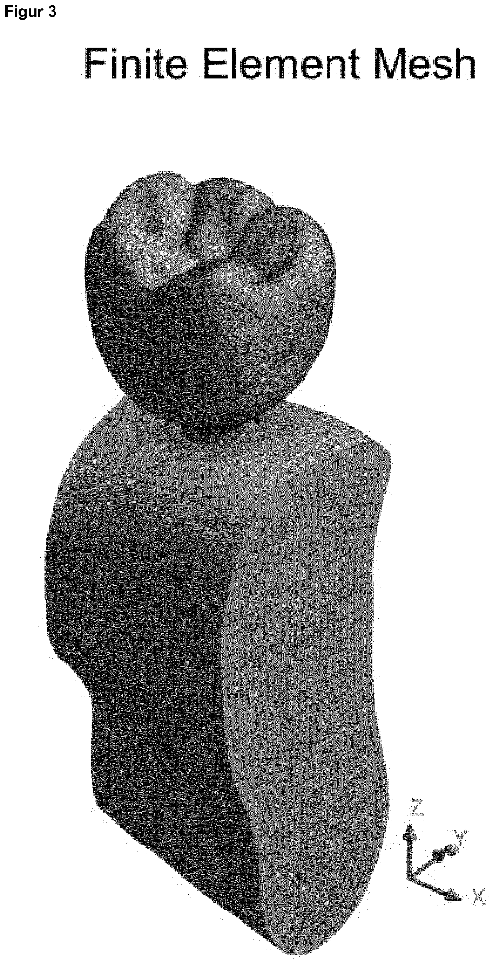

Anschließend wurde ein vorwiegend hexaedrisches Finite-Elemente Netz mit den Elementtypen Tet10, Hex20, Wed15 und pyr13 mit Verfeinerung im Bereich der Kontakte erstellt. Dieses bestand im Falle des C-1-Modells aus 137425 Elementen und 498543 Knoten und im Falle des C-2-Modells aus 143210 Elementen und 530334 Knoten (Figur 3).

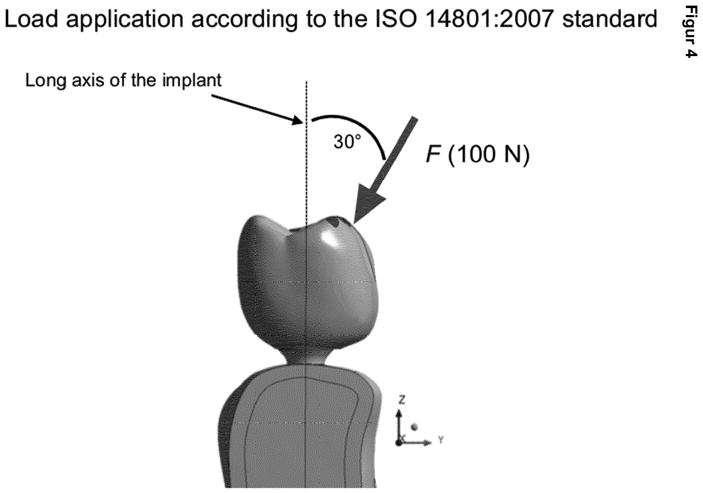

Anschließend wurde entsprechend der ISO 14801:2007 eine Last von 100 N im 30°-Winkel zur Längsachse des Implantates in die Implantatkrone eingeleitet (Figur 4) und die Oberflächenkontaktdrücke und die Deformationen im periimplantären Knochen analysiert.

### Ergebnisse:

Die Ergebnisse der Oberflächenkontaktdrücke und Deformationen im periimplantären Knochen bei Belastung der Implantate sind in Tabelle 3 zusammengefasst.

**Tabelle 3: Zusammenfassung der Ergebnisse für die maximale Gesamtverformung des periimplantären Knochens und die maximalen Kontaktdrücke am Implantat-Knochen-Interface bei variablen E-Moduli der Endloskohlefaser-verstärkten PEEK-Anteile.**

| | Einkomponenten-Implantat (1-C) | | Zweikomponenten-Implantat (2-C) | |
|---|---|---|---|---|
| E-Modul des Anteils aus 55-CFR-PEEK | Maximale Gesamtverformung des Knochens | Maximaler Kontaktdruck am Implantat-Knochen-Interface | Maximale Gesamtverformung des Knochens | Maximaler Kontaktdruck am Implantat-Knochen-Interface |
| [MPa] | [µm] | [MPa] | [µm] | [MPa] |
| 40 | 9,4 | 51,9 | 11 | 56,7 |

### Schlussfolgerungen:

Überraschend zeigte sich, dass ein erfindungsgemäßes Einkomponenten-Implantat (1-C) im Vergleich zu einem Zweikomponenten-Implantat (2-C) gemäß der Erfindung WO 2014/198421 A1 bei Belastung hinsichtlich seiner mechanischen Auswirkungen auf den Knochen ein vorteilhafte Eigenschaften aufweist. Bei Verwendung des 1-C Implantats kam es zu einer geringeren maximalen Gesamtverformung des Knochens und der maximale Kontaktdruck am Implantat-Knochen-Interface war gegenüber einem 2-C Implantat verringert.

## Patentansprüche

1. Verfahren zur Herstellung eines Implantats mit einem Elastizitätsmodul E von 10-70 GPa, umfassend
a. die Erfassung von Patientendaten bzal der Ilmgebung in die ein Implantat eingesetzt werden soll, wobei die erfassten Patientendaten
i. die Krafteinwirkung bei mechanischer Belastung,
ii. die Knochendichte und/oder
iii. die räumliche Struktur der Umgebung umfassen,
b. die Erstellung eines Modells für ein individualisiertes Implantat auf Basis der erfassten Patientendaten, wobei
i. eine dreidimensionale Struktur des Implantats,
ii. eine Materialzusammensetzung umfassend einen thermoplastischen Kunststoff, verstärkende Langfasern und optional Compoundzusätze, und
iii. eine multidirektionale Anordnung der verstärkenden Langfasern und optional der Compoundzusätze berechnet werden,
c. die Herstellung des individualisierten Implantats auf Basis des berechneten Modells mittels 3D-Druck und/oder Lasersintern.

2. System zur Herstellung eines Implantats mit einem Elastizitätsmodul E von 10-70 GPa gemäß dem Verfahren nach Anspruch 1, umfassend
a. eine Vorrichtung zur Erfassung von Patientendaten bzgl. der Umgebung, in die eingesetzt werden soll, wobei die erfassten Patientendaten
i. die Krafteinwirkung bei mechanischer Belastung,
ii. die Knochendichte und/oder
iii. die räumliche Struktur der Umgebung umfassen,
b. ein Computerprogramm zur Erstellung eines Modells für ein individualisiertes Implantat auf Basis der erfassten Patientendaten, wobei
i. eine dreidimensionale Struktur des Implantats,
ii. eine Materialzusammensetzung umfassend einen thermoplastischen Kunststoff, verstärkende Langfasern und optional Compoundzusätze, und
iii. eine multidirektionale Anordnung der verstärkenden Langfasern und optional der Compoundzusätze berechnet werden, und
c. eine Vorrichtung zur Herstellung des individualisierten Implantats auf Basis des berechneten Modells mittels 3D-Druck und/oder Lasersintern.

3. Vorrichtung zur Herstellung eines Implantats mit einem Elastizitätsmodul E von 10-70 GPa, nach dem Verfahren gemäß Anspruch 1, umfassend
a. eine Einrichtung zur Erfassung von Patientendaten bzgl. der Umgebung, in die eingesetzt werden soll, wobei die erfassten Patientendaten
i. die Krafteinwirkung bei mechanischer Belastung,
ii. die Knochendichte und/oder
iii. die räumliche Struktur der Umgebung umfassen,
b. eine Computereinrichtung zur Erstellung eines Modells für ein individualisiertes Implantat auf Basis der erfassten Patientendaten, wobei
i. eine dreidimensionale Struktur des Implantats,
ii. eine Materialzusammensetzung umfassend einen thermoplastischen Kunststoff, verstärkende Langfasern und optional Compoundzusätze, und
iii. eine multidirektionale Anordnung der verstärkenden Langfasern und optional der Compoundzusätze berechnet werden, und
c. eine Einrichtung zur Herstellung des individualisierten Implantats auf Basis des berechneten Modells mittels 3D-Druck und/oder Lasersintern

## Claims

1. A method for producing an implant having a modulus of elasticity E of 10-70 GPa, including
a. the collection of patient data regarding the environment into which an implant is to be inserted, wherein the collected patient data includes
i. the force application under mechanical load,
ii. the bone density, and/or
iii. the spatial structure of the environment,
b. the creation of a model for a customized implant based on the collected patient data, wherein
i. a three-dimensional structure of the implant,
ii. a material composition including a thermoplastic plastic, reinforcing long fibers and optionally compound additives, and
iii. a multidirectional arrangement of the reinforcing long fibers and optionally of the compound additives are calculated,
c. the production of the customized implant based on the calculated model by 3D printing and/or laser sintering.

2. A system for producing an implant having a modulus of elasticity E of 10-70 GPa according to the method of claim 1, including
a. a device for collecting patient data regarding the environment into which the implant is to be inserted, wherein the collected patient data includes
i. the force application under mechanical load,
ii. the bone density, and/or
iii. the spatial structure of the environment,
b. a computer program for creating a model for a customized implant based on the collected patient data, wherein
i. a three-dimensional structure of the implant,
ii. a material composition including a thermoplastic plastic, reinforcing long fibers and optionally compound additives, and
iii. a multidirectional arrangement of the reinforcing long fibers and optionally of the compound additives are calculated, and
c. a device for producing the customized implant based on the calculated model by 3D printing and/or laser sintering.

3. A device for producing an implant having a modulus of elasticity E of 10-70 GPa according to the method of claim 1, including
a. a unit for collecting patient data regarding the environment into which the implant is to be inserted, wherein the collected patient data includes
i. the force application under mechanical load,
ii. the bone density, and/or
iii. the spatial structure of the environment,
b. a computer unit for creating a model for a customized implant based on the collected patient data, wherein
i. a three-dimensional structure of the implant,
ii. a material composition including a thermoplastic plastic, reinforcing long fibers and optionally compound additives, and
iii. a multidirectional arrangement of the reinforcing long fibers and optionally of the compound additives are calculated, and
c. a unit for producing the customized implant based on the calculated model by 3D printing and/or laser sintering.

## Revendications

1. Procédé de fabrication d'un implant avec un module d'élasticité E de 10 à 70 GPa, comprenant
a. l'acquisition de données de patient concernant l'environnement immédiat dans lequel un implant doit être mis en œuvre, dans lequel les données de patient acquises comprennent
i. l'action de force lors d'une sollicitation mécanique,
ii. la densité osseuse et/ou
iii. la structure spatiale de l'environnement immédiat,
b. la génération d'un modèle pour un implant individualisé sur la base des données de patient acquises, dans lequel
i. une structure tridimensionnelle de l'implant,
ii. une composition en matériaux comprenant une matière plastique thermoplastique, des fibres longitudinales de renforcement et facultativement des additifs de composé, et
iii. une disposition multidirectionnelle des fibres longitudinales de renforcement et facultativement des additifs de composé sont calculées,
c. la fabrication de l'implant individualisé sur la base du modèle calculé au moyen d'une impression 3D et/ou d'un frittage au laser.

2. Système de fabrication d'un implant avec un module d'élasticité E de 10 à 70 GPa selon le procédé selon la revendication 1, comprenant
a. un dispositif d'acquisition de données de patient concernant l'environnement immédiat dans lequel un implant doit être mis en œuvre, dans lequel les données de patient acquises comprennent
i. l'action de force lors d'une sollicitation mécanique,
ii. la densité osseuse et/ou
iii. la structure spatiale de l'environnement immédiat,
b. un programme informatique de génération d'un modèle pour un implant individualisé sur la base des données de patient acquises, dans lequel
i. une structure tridimensionnelle de l'implant,
ii. une composition en matériaux comprenant une matière plastique thermoplastique, des fibres longitudinales de renforcement et facultativement des additifs de composé, et
iii. une disposition multidirectionnelle des fibres longitudinales de renforcement et facultativement des additifs de composé sont calculées, et
c. un dispositif de fabrication de l'implant individualisé sur la base du modèle calculé au moyen d'une impression 3D et/ou d'un frittage au laser.

3. Dispositif de fabrication d'un implant avec un module d'élasticité E de 10 à 70 GPa selon le procédé selon la revendication 1, comprenant
a. un dispositif d'acquisition de données de patient concernant l'environnement immédiat, dans lequel un implant doit être mis en œuvre, dans lequel les données de patient acquises comprennent
i. l'action de force lors d'une sollicitation mécanique,
ii. la densité osseuse et/ou
iii. la structure spatiale de l'environnement immédiat,
b. un dispositif informatique de fabrication d'un modèle pour un implant individualisé sur la base des données de patient acquises, dans lequel
i. une structure tridimensionnelle de l'implant,
ii. une composition en matériaux comprenant une matière plastique thermoplastique, des fibres longitudinales de renforcement et facultativement des additifs de composé, et
iii. une disposition multidirectionnelle des fibres longitudinales de renforcement et facultativement des additifs de composé sont calculées, et
c. un dispositif de fabrication de l'implant individualisé sur la base du modèle calculé au moyen d'une impression 3D et/ou d'un frittage au laser.
